(19) Europäisches Patentamt
European Patent Office
Office européen des brevets

(11) **EP 2 369 337 A2**

(12) **EUROPEAN PATENT APPLICATION**

(43) Date of publication:
**28.09.2011 Bulletin 2011/39**

(51) Int Cl.:
***G01N 33/22*** *(2006.01)*

(21) Application number: **11151331.3**

(22) Date of filing: **19.01.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.03.2010 JP 2010069583**

(71) Applicant: **Yamatake Corporation Tokyo 100-6419 (JP)**

(72) Inventor: **Ooishi, Yasuharu Tokyo 100-6419 (JP)**

(74) Representative: **Tomlinson, Kerry John Dehns St Bride's House 10 Salisbury Square London EC4Y 8JD (GB)**

(54) **Calorific value calculation formula creation system, method of creating calorific value calculation formula, calorific value measurement system, and method of measuring calorific value**

(57) There is provided a calorific value calculation formula creation system (21). The system (21) includes: a container (101) into which a mixed gas including a plurality of gases are injected; a heater element (61) that is provided in the container; a measurement mechanism (10) that corrects a drift of a resistance value of the heater element based on the ratio of a first resistance value ($R_{H0}$) of the heater element when a first temperature ($T_{H0}$) of the heater element is approximate to an atmosphere temperature ($T_I$) and a second resistance value ($R_{H1}$) of the heater element when a second temperature ($T_{H1}$) of the heater element is more than the atmosphere temperature ($T_I$), and measures radiation coefficients ($M_A(T_{H1})$, $M_B(T_{H2})$...) of the plurality of gases at each of a plurality of heating temperatures of the heater element; and a formula creation module (302) that creates a calorific value calculation formula based on a given calorific value (Q) of the mixed gas and the radiation coefficients ($M_A(T_{H1})$, $M_B(T_{H2})$...), wherein the calorific value calculation formula is represented by the radiation coefficients at each of the plurality of heating temperatures that are set as independent variables, and a calorific value (Q) of the mixed gas that is set as dependent variable.

*FIG. 6*

EP 2 369 337 A2

**Description**

Technical Field

The present invention relates to a gas examination technique, and more particularly, to a calorific value calculation formula creation system, a method of creating a calorific value calculation formula, a calorific value measurement system, and a method of measuring a calorific value.

Related Art

In the related art, when the calorific value of a mixed gas is calculated, it is necessary to analyze the components of the mixed gas using, for example, an expensive gas chromatography device. In addition, a method has been proposed which measures the thermal conductivity of a mixed gas and the speed of sound in the mixed gas to calculate the percentage of components, such as methane ($CH_4$), propane ($C_3H_8$), nitrogen ($N_2$), and carbon dioxide ($CO_2$), included in the mixed gas, thereby calculating the calorific value of the mixed gas (for example, see JP-T-2004-514138).

SUMMARY

According to one or more illustrative aspects of the present invention, there is provided a calorific value calculation formula creation system (21). The system includes: a container (101) into which a mixed gas including a plurality of gases are injected; a heater element (61) that is provided in the container; a measurement mechanism (10) that corrects a drift of a resistance value of the heater element based on the ratio of a first resistance value ($R_{H0}$) of the heater element when a first temperature ($T_{H0}$) of the heater element is approximate to an atmosphere temperature ($T_I$) and a second resistance value ($R_{H1}$) of the heater element when a second temperature ($T_{H1}$) of the heater element is more than the atmosphere temperature ($T_1$), and measures radiation coefficients ($M_A(T_{H1})$, $M_B(T_{H2})$...) of the plurality of gases at each of a plurality of heating temperatures of the heater element; and a formula creation module (302) that creates a calorific value calculation formula based on a given calorific value (Q) of the mixed gas and the radiation coefficients ($M_A(T_{H1})$, $M_B(T_{H2})$...), wherein the calorific value calculation formula is represented by the radiation coefficients at each of the plurality of heating temperatures that are set as independent variables, and a calorific value (Q) of the mixed gas that is set as dependent variable.

According to one or more illustrative aspects of the present invention, there is provided a method of creating a calorific value calculation formula. The method includes: (a) preparing a mixed gas including a plurality of gases; (b) correcting a drift of a resistance value of a heater element (61) based on the ratio of a first resistance value ($R_{H0}$) of the heater element when a first temperature ($T_{H0}$) of the heater element is approximate to an atmosphere temperature ($T_1$) and a second resistance value ($R_{H1}$) of the heater element when a second temperature ($T_{H1}$) of the heater element is more than the atmosphere temperature ($T_1$); (c) measuring radiation coefficients ($M_A(T_{H1})$, $M_B(T_{H2})$...) of the plurality of gases at each of a plurality of heating temperatures of the heater element; and (e) creating a calorific value calculation based on a given calorific value (Q) of the mixed gas and the radiation coefficients ($M_A(T_{H1)}$, $M_B(T_{H2})$...), wherein the calorific value calculation formula is represented by the radiation coefficients at each of the plurality of heating temperatures that are set as independent variables, and a calorific value (Q) of the mixed gas that is set as dependent variable.

According to one or more illustrative aspects of the present invention, there is provided a calorific value measurement system. The system includes: a container (101) into which a mixed gas including a plurality of gases are injected, wherein a calorific value of the mixed gas is unknown before measurement; a heater element that (61) is provided in the container; a measurement mechanism (10) that corrects a drift of a resistance value of the heater element based on the ratio of a first resistance value ($R_{H0}$) of the heater element when a first temperature ($T_{H0}$) of the heater element is approximate to an atmosphere temperature ($T_1$) and a second resistance value ($R_{H1}$) of the heater element when a second temperature ($T_{H1}$) of the heater element is more than the atmosphere temperature ($T_1$), and measures radiation coefficients ($M_{I1}$, $M_{I2}$...) of the mixed gas at a plurality of heating temperatures of the heater element; a formula storage device that stores a calorific value calculation formula, wherein the calorific value calculation formula is represented by the radiation coefficients ($M_{I1}$, $M_{I2}$...) of the mixed gas that are set as independent variables, and a calorific value (Q) of the mixed gas that is set as dependent variable; and a calorific value calculation module that substitutes the measured radiation coefficients into the radiation coefficients of the calorific value calculation formula to calculate the calorific value of the mixed gas.

According to one or more illustrative aspects of the present invention, there is provided a method of measuring a calorific value. The method includes: a) preparing a mixed gas including a plurality of gases, wherein a calorific value of the mixed gas is unknown before measurement; b) correcting a drift of a resistance value of a heater element (61) based on the ratio of a first resistance value ($R_{H0}$) of the heater element when a first temperature ($T_{H0}$) of the heater element is approximate to an atmosphere temperature ($T_1$) and a second resistance value ($R_{H1}$) of the heater element when a second temperature ($T_{H1}$) of the heater element is more than the atmosphere temperature ($T_1$); (c) measuring radiation coefficients ($M_{I1}, M_{I2},..$) of the mixed gas at a plurality of heating temperatures of the heater element; (d) preparing a calorific value calculation formula, wherein the calorific value calculation formula is represented by the radiation coefficients ($M_{I1}, M_{I2}...$) of the mixed gas that are set as independent variables, and a calorific value ($Q$) of the mixed gas that is set as dependent variable; and (e) substituting the measured radiation coefficients into the radiation coefficients of the calorific value calculation formula to calculate the calorific value of the mixed gas.

According to one or more illustrative aspects of the present invention, there is provided a property measurement system. The system includes: a container (101) into which a gas is injected; a heater element (61) that is provided in the container; and a measurement mechanism (10) that corrects a drift of a resistance value of the heater element based on the ratio of a first resistance value ($R_{H0}$) of the heater element when a first temperature ($T_{H0}$) of the heater element is approximate to an atmosphere temperature ($T_1$) and a second resistance value ($R_{H1}$) of the heater element when a second temperature ($T_{H1}$) of the heater element is more than the atmosphere temperature ($T_1$), and measures a radiation coefficient ($M$) of the gas.

According to one or more illustrative aspects of the present invention, there is provided a method of measuring a property. The method includes: (a) preparing a gas; (b) correcting a drift of a resistance value of a heater element (61) based on the ratio of a first resistance value ($R_{H0}$) of the heater element when a first temperature ($T_{H0}$) of the heater element is approximate to an atmosphere temperature ($T_1$) and a second resistance value ($R_{H1}$) of the heater element when a second temperature ($T_{H1}$) of the heater element is more than the atmosphere temperature ($T_1$); and (c) measuring a radiation coefficient of the gas.

BRIEF DESCRIPTION OF THE DRAWINGS

Fig. 1 is a perspective view illustrating a microchip according to a first embodiment of the invention;
Fig. 2 is a cross-sectional view illustrating the microchip according to the first embodiment of the invention taken along the line II-II of Fig. 1;
Fig. 3 is a circuit diagram illustrating a heater element according to the first embodiment of the invention;
Fig. 4 is a circuit diagram illustrating a temperature measurement element according to the first embodiment of the invention;
Fig. 5 is a graph illustrating the relationship between the radiation coefficient of gas and the temperature of the heater element according to the first embodiment of the invention;
Fig. 6 is a first schematic diagram illustrating a gas property value measurement system according to the first embodiment of the invention;
Fig. 7 is a second schematic diagram illustrating the gas property value measurement system according to the first embodiment of the invention;
Fig. 8 is a flowchart illustrating a method of creating a calorific value calculation formula according to the first embodiment of the invention;
Fig. 9 is a schematic diagram illustrating a gas property value measurement system according to a second embodiment of the invention;
Fig. 10 is a graph illustrating the relationship between thermal conductivity and a radiation coefficient according to the second embodiment of the invention;
Fig. 11 is a schematic diagram illustrating a gas property value measurement system according to a third embodiment of the invention;
Fig. 12 is a graph illustrating the relationship between the concentration and the radiation coefficient of gas according to the third embodiment of the invention;
Fig. 13 is a schematic diagram illustrating a gas property value measurement system according to a fourth embodiment of the invention;
Fig. 14 is a flowchart illustrating a method of measuring a calorific value according to the fourth embodiment of the invention;
Fig. 15 is a table illustrating the composition and calorific value of a sample mixed gas according to Example 1 of the embodiment of the invention;
Fig. 16 is a graph illustrating the calculated calorific value and the true calorific value of the sample mixed gas according to Example 1 of the embodiment of the invention; and
Fig. 17 is a graph illustrating the relationship between the true calorific value and the calculated calorific value of the sample mixed gas according to Example 1 of the embodiment of the invention.

DETAILED DESCRIPTION OF EXEMPLARY EMBODIMENTS

Hereinafter, exemplary embodiments of the invention will be described. In the following description of the drawings, the same or similar components are denoted by the same or similar reference numerals. However, the drawings are schematically illustrated. Therefore, for example, detailed dimensions need to be determined in the light of the following description. In addition, in the drawings, the same components may have different dimensions or ratios.

(First embodiment)

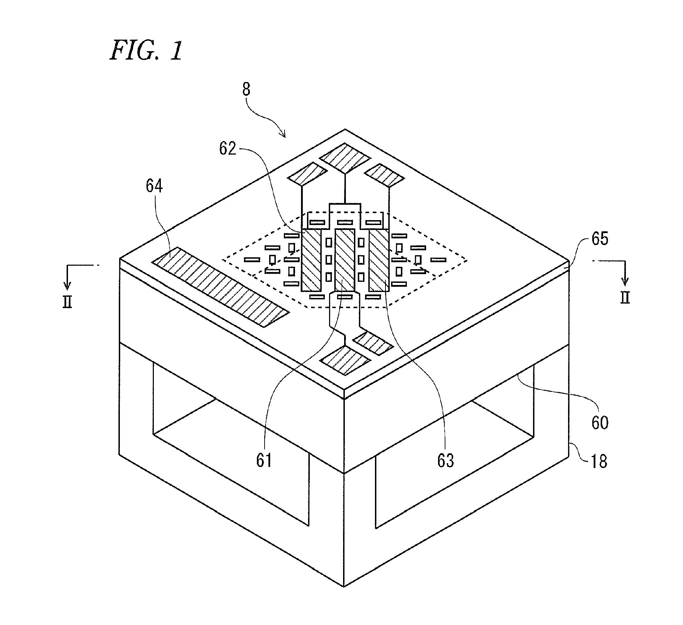

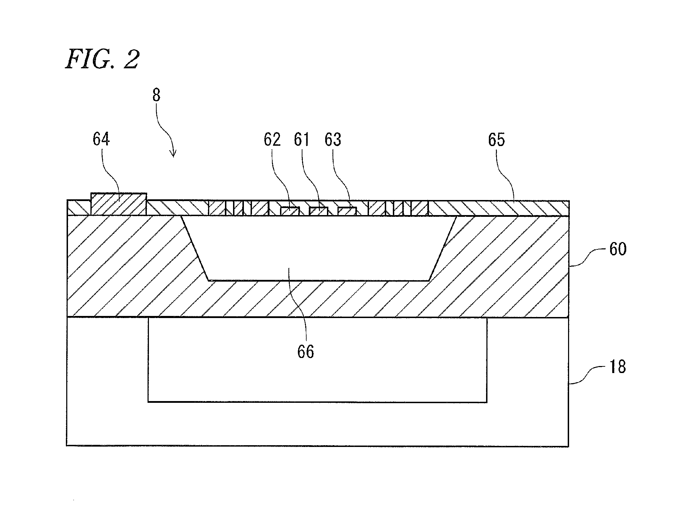

First, a microchip 8 used in a gas property value measurement system according to a first embodiment will be described with reference to Fig. 1, which is a perspective view, and Fig. 2, which is a cross-sectional view taken along the line II-II. The microchip 8 includes a substrate 60 having a cavity 66 provided therein and an insulating film 65 that is provided on the substrate 60 so as to cover the cavity 66. The thickness of the substrate 60 is, for example, 0.5 mm. In addition, the substrate 60 has, for example, a size of about 1.5 mm x 1.5 mm. A portion of the insulating film 65 covering the cavity 66 is a heat insulating diaphragm. The microchip 8 further includes a heater element 61 that is provided in the diaphragm portion of the insulating film 65, first and second temperature measurement elements 62 and 63 that are provided in the diaphragm portion of the insulating film 65 with the heater element 61 interposed therebetween, and a heat-retaining element 64 that is provided on the substrate 60.

The heater element 61 is arranged at the center of the diaphragm portion of the insulating film 65 covering the cavity 66. The heater element 61 is, for example, a resistor and is supplied with power to generate heat, thereby heating an atmosphere gas coming into contact with the heater element 61. The first temperature measurement element 62 and the second temperature measurement element 63 are, for example, resistors and detect the temperature of the atmosphere gas before the heater element 61 generates heat. In addition, only the first temperature measurement element 62 or the second temperature measurement element 63 may be used to detect the gas temperature. Alternatively, the average value of the gas temperature detected by the first temperature measurement element 62 and the gas temperature detected by the second temperature measurement element 63 may be used as the gas temperature. Next, an example in which the average value of the gas temperature values detected by the first temperature measurement element 62 and the second temperature measurement element 63 is used as the gas temperature will be described, but the invention is not limited thereto.

The heat-retaining element 64 is, for example, a resistor and is supplied with power to generate heat, thereby maintaining the temperature of the substrate 60 to be constant. The substrate 60 may be made of, for example, silicon (Si). The insulating film 65 may be made of, for example, silicon oxide ($SiO_2$). The cavity 66 is formed by, for example, anisotropic etching. The heater element 61, the first temperature measurement element 62, the second temperature measurement element 63, and the heat-retaining element 64 may be made of, for example, platinum (Pt) and may be formed by, for example, a lithography method.

The microchip 8 is fixed to a container, such as a chamber filled up with an atmosphere gas, with a heat insulating member 18 which is provided on the bottom of the microchip 8 interposed therebetween. When the microchip 8 is fixed to, for example, the chamber with the heat insulating member 18 interposed therebetween, the temperature of the microchip 8 is less likely to be affected by a variation in the temperature of the inner wall of the chamber. For example, the thermal conductivity of the heat insulating member 18 made of glass is equal to or less than 1.0 W/(m·K).

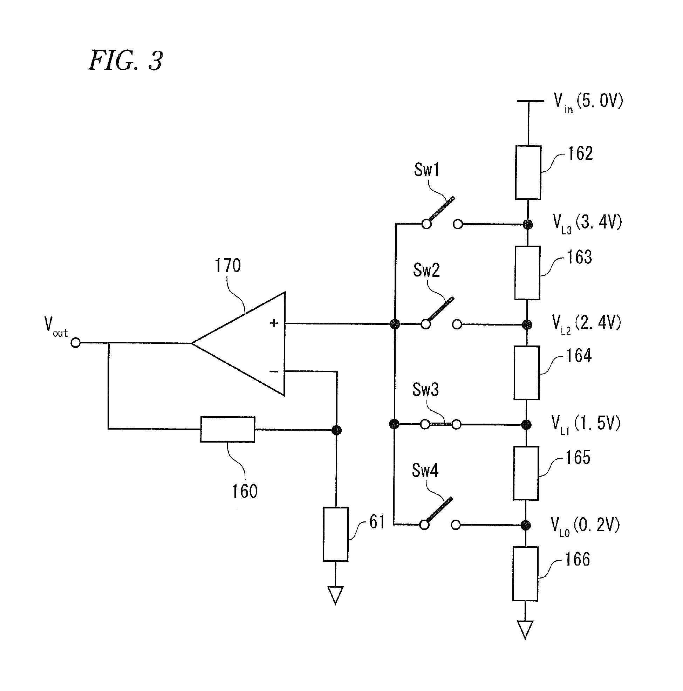

As shown in Fig. 3, the heater element 61 has one end that is electrically connected to, for example, a negative (-) input terminal of an operational amplifier 170 and the other end that is connected to the ground. A resistive element 160 is connected in parallel to the negative input terminal and an output terminal of the operational amplifier 170. A positive (+) input terminal of the operational amplifier 170 is electrically connected between a resistive element 162 and a resistive element 163 connected in series to each other, between the resistive element 163 and a resistive element 164 connected in series to each other, between the resistive element 164 and a resistive element 165 connected in series to each other, or between the resistive element 165 and a resistive element 166 connected in series to each other. The resistance values of the resistive elements 162 to 166 are appropriately determined. For example, a voltage Vin of 5.0 V is applied to one end of the resistive element 162, a voltage $V_{L3}$ of, for example, 3.4 V is generated between the resistive element 163 and the resistive element 162. In addition, a voltage $V_{L2}$ of, for example, 2.4 V is generated between the resistive element 164 and the resistive element 163 and a voltage $V_{L1}$ of, for example, 1.5 V is generated between the resistive element 165 and the resistive element 164. A voltage $V_{L0}$ of, for example, 0.2 V is generated between the resistive element 166 and the resistive element 165.

A switch SW1 is provided between the positive input terminal of the operational amplifier and a point between the resistive element 162 and the resistive element 163, and a switch SW2 is provided between the positive input terminal of the operational amplifier and a point between the resistive element 163 and the resistive element 164. In addition, a switch SW3 is provided between the positive input terminal of the operational amplifier and a point between the resistive element 164 and the resistive element 165, and a switch SW4 is provided between the ground terminal of the resistive element 165 and the positive input terminal of the operational amplifier.

When a voltage $V_{L3}$ of 3.4 V is applied to the positive input terminal of the operational amplifier 170, only the switch SW1 is turned on and the switches SW2, SW3, and SW4 are turned off. When a voltage $V_{L2}$ of 2.4 V is applied to the positive input terminal of the operational amplifier 170, only the switch SW2 is turned on and the switches SW1, SW3, and SW4 are turned off. When a voltage $V_{L1}$ of 1.5 V is applied to the positive input terminal of the operational amplifier 170, only the switch SW3 is turned on and the switches SW1, SW2, and SW4 are turned off. When a voltage $V_{L0}$ of 0.2 V is applied to the positive input terminal of the operational amplifier 170, only the switch SW4 is turned on and the switches SW1, SW2, and SW3 are turned off.

Therefore, it is possible to turn on or off the switches SW1, SW2, SW3, and SW4 to apply any one of four-stage voltages to the negative input terminal of the operational amplifier 170. As a result, it is possible to turn on or off the switches SW1, SW2, SW3, and SW4 to set a voltage for determining the temperature of the heater element 61 in four stages. It is assumed that the temperature of the heater element 61 when a voltage $V_{L0}$ of 0.2 V is applied to the positive input terminal of the operational amplifier 170 is $T_{H0}$ and the temperature of the heater element 61 when a voltage $V_{L1}$ of 1.5 V is applied to the positive input terminal of the operational amplifier 170 is $T_{H1}$. In addition, it is assumed that the temperature of the heater element 61 when a voltage $V_{L2}$ of 2.4 V is applied to the positive input terminal of the operational amplifier 170 is $T_{H2}$ and the temperature of the heater element 61 when a voltage $V_{L3}$ of 3.4 V is applied to the positive input terminal of the operational amplifier 170 is $T_{H3}$.

When a low voltage $V_{L0}$ of 0.2 V is applied to the positive input terminal of the operational amplifier 170, the temperature $T_{H0}$ of the heater element 61 is substantially equal to that when no voltage is applied to the positive input terminal of the operational amplifier 170. Therefore, the temperature $T_{H0}$ of the heater element 61 is equal or approximate to the temperature of the atmosphere gas. In contrast, when the voltages $V_{L1}$, $V_{L2}$, and $V_{L3}$ are applied to the positive input terminal of the operational amplifier 170, the temperatures $T_{H1}$, $T_{H2}$, and $T_{H3}$ of the heater element 61 are higher than that when no voltage is applied to the positive input terminal of the operational amplifier 170, and are higher than the temperature of the atmosphere gas.

In the heater element 61 shown in Figs. 1 and 2, the resistance value of the heater element 61 varies depending on the temperature thereof. The relationship between the temperature $T_H$ of the heater element 61 and the resistance value $R_H$ of the heater element 61 is represented by the following Expression (1):

$$R_H = R_{STD} \times [1 + \alpha(T_H - T_{STD})], \qquad \ldots(1)$$

where $T_{STD}$ indicates the standard temperature of the heater element 61 and is, for example, 20°C, $R_{STD}$ indicates the resistance value of the heater element 61 which is measured at the standard temperature $T_{STD}$ in advance, and $\alpha$ indicates a primary resistance-temperature coefficient).

The resistance value $R_H$ of the heater element 61 is calculated from the driving power $P_H$ of the heater element 61 and the on-current $I_H$ of the heater element 61 by the following Expression (2):

$$R_H = P_H / I_H{}^2. \qquad \ldots(2)$$

Alternatively, the resistance value $R_H$ of the heater element 61 is calculated from a voltage $V_H$ applied to the heater element 61 and the on-current $I_H$ of the heater element 61 by the following Expression (3):

$$R_H = V_H / I_H. \qquad \ldots(3)$$

The temperature $T_H$ of the heater element 61 is stabilized when the heater element 61 and the atmosphere gas are in a thermal equilibrium state. The thermal equilibrium state means that the generation of heat from the heater element 61 and the heat dissipation of the heater element 61 to the atmosphere gas are in equilibrium. In the equilibrium state, as shown in the following Expression (4), the radiation coefficient $M_1$ of the atmosphere gas is obtained by dividing the driving power $P_H$ of the heater element 61 by the difference $\Delta T_H$ between the temperature $T_H$ of the heater element 61 and the temperature $T_1$ of the atmosphere gas:

$$M_I = P_H/(T_H-T_I)$$
$$= P_H/\Delta T_H. \qquad \ldots(4)$$

The unit of the radiation coefficient $M_1$ is, for example, W/°C.

With the Expression (1), the temperature $T_H$ of the heater element 61 is represented by the following Expression (5):

$$T_H = (1/\alpha)\times[(R_H/R_{STD})-1]+T_{STD}. \qquad \ldots(5)$$

Therefore, the difference $\Delta T_H$ between the temperature $T_H$ of the heater element 61 and the temperature $T_1$ of the atmosphere gas is represented by the following Expression (6):

$$\Delta T_H = (1/\alpha)\times[(R_H/R_{STD})-1]+T_{STD}-T_I. \qquad \ldots(6)$$

Since the on-current $I_H$ of the heater element 61, and the driving power $P_H$ or the voltage $V_H$ can be measured, it is possible to calculate the resistance value $R_H$ of the heater element 61 from Expression (2) or Expression (3). In addition, the temperature $T_1$ of the atmosphere gas can be measured by the first temperature measurement element 62 and the second temperature measurement element 63 shown in Fig. 1. Therefore, it is possible to calculate the radiation coefficient $M_1$ of the atmosphere gas from the following Expression 7 using the microchip 8 shown in Figs. 1 and 2:

$$M_I = P_H/\Delta T_H$$
$$= P_H/[(1/\alpha)\times[(R_H/R_{STD})-1]+T_{STD}-T_I]. \qquad \ldots(7)$$

However, in some cases, the resistance value $R_{STD}$ of the heater element 61 when the temperature of the heater element 61 is the standard temperature $T_{STD}$ drifts from the value which is measured in advance, for example, over time. Therefore, when the resistance value $R_{STD}$ which is measured in advance is used to calculate the radiation coefficient $M_1$ from Expression (7), an error is likely to occur.

With Expression (6), the resistance value $R_{H1}$ of the heater element 61 when the temperature of the heater element 61 is $T_{H1}$ is represented by the following Expression (8):

$$R_{H1} = [(\Delta T_{H1}-T_{STD}+T_I)\alpha+1]\times R_{STD}. \qquad \ldots(8)$$

In addition, the resistance value $R_{H0}$ of the heater element 61 when the temperature of the heater element 61 is $T_{H0}$ is represented by the following Expression (9):

$$R_{H0} = [(\Delta T_{H0}-T_{STD}+T_I)\alpha+1]\times R_{STD}. \qquad \ldots(9)$$

As shown in the following Expression (10), when the temperature $T_{H0}$ of the heater element 61 is approximate to the temperature $T_1$ of the atmosphere gas, the resistance value $R_{H0}$ of the heater element 61 when the temperature of the heater element 61 is $T_{H0}$ is represented by the following Expression (11):

$$T_{H0} \approx T_I, \qquad \ldots(10)$$

$$R_{H0} = [(-T_{STD}+T_I)\alpha+1] \times R_{STD}. \qquad \dots(11)$$

The ratio of the resistance value $R_{H1}$ of the heater element 61 to the resistance value $R_{H0}$ of the heater element 61 is represented by the following Expression (12):

$$R_{H1}/R_{H0} = [[(\Delta T_{H1}-T_{STD}+T_I)\alpha+1] \times R_{STD}]/[[(-T_{STD}+T_I)\alpha+1] \times R_{STD}]$$

$$= [[(\Delta T_{H1}-T_{STD}+T_I)\alpha+1]]/[[(-T_{STD}+T_I)\alpha+1]]. \qquad \dots(12)$$

With Expression (12), the difference $\Delta T_{H1}$ between the temperature $T_{H1}$ of the heater element 61 and the temperature $T_1$ of the atmosphere gas is represented by the following Expression (13):

$$\Delta T_{H1} = [[(-T_{STD}+T_I)\alpha+1] \times R_{H1}/R_{H0}-1] \times (1/\alpha)+T_{STD}-T_I$$

$$= [(-T_{STD}+T_I)+1/\alpha] \times R_{H1}/R_{H0}-1/\alpha+T_{STD}-T_I$$

$$= (-T_{STD}+T_I)R_{H1}/R_{H0}+(1/\alpha) \times R_{H1}/R_{H0}-1/\alpha+T_{STD}-T_I$$

$$= (1/\alpha) \times (R_{H1}/R_{H0}-1)+(T_I+T_{STD})R_{H1}/R_{H0}+T_{STD}-T_I$$

$$= (1/\alpha) \times (R_{H1}/R_{H0}-1)-T_{STD}(R_{H1}/R_{H0}-1)+T_I R_{H1}/R_{H0}-T_I$$

$$= (1/\alpha-T_{STD}) \times (R_{H1}/R_{H0}-1)+T_I R_{H1}/R_{H0}-T_I$$

$$= (1/\alpha-T_{STD}) \times (R_{H1}/R_{H0}-1)+T_I(R_{H1}/R_{H0}-1)$$

$$= (1/\alpha-T_{STD}+T_I) \times (R_{H1}/R_{H0}-1). \qquad \dots(13)$$

With Expression (13), when the temperature of the heater element 61 is $T_{H1}$, the radiation coefficient $M_{I1}$ of the atmosphere gas is represented by the following Expression (14):

$$M_{I1} = P_{H1}/\Delta T_{H1}$$

$$= P_{H1}/[(1/\alpha-T_{STD}+T_I) \times (R_{H1}/R_{H0}-1)]. \qquad \dots(14)$$

When the radiation coefficient $M_{I1}$ of the atmosphere gas is calculated by Expression (14), it is not necessary to use the resistance value $R_{STD}$ of the heater element 61 that is measured at the standard temperature $T_{STD}$ in advance. Therefore, when Expression (14) is used, it is possible to correct the drift of the resistance value of the heater element 61, which is a variation in the resistance value $R_{SID}$ of the heater element 61 at the standard temperature $T_{STD}$, and accurately calculate the radiation coefficient $M_{I1}$ of the atmosphere gas.

When the temperature of the heater element 61 is $T_{H2}$, the radiation coefficient $M_{I2}$ of the atmosphere gas is represented by the following Expression (15):

$$M_{I2} = P_{H2}/\Delta T_{H2}$$

$$= P_{H2}/[(1/\alpha-T_{STD}+T_I) \times (R_{H2}/R_{H0}-1)]. \qquad \dots(15)$$

When the temperature of the heater element 61 is $T_{H3}$, the radiation coefficient $M_{I3}$ of the atmosphere gas is represented by the following Expression (16):

$$M_{I3} = P_{H3}/\Delta T_{H3}$$

$$= P_{H3}/[(1/\alpha-T_{STD}+T_I) \times (R_{H3}/R_{H0}-1)]. \qquad \dots(16)$$

Since the heat-retaining element 64 maintains the temperature of the substrate 60 to be constant, the temperature of the atmosphere gas in the vicinity of the microchip 8 before the heater element 61 generates heat is approximate to the constant temperature of the substrate 60. Therefore, a variation in the temperature of the atmosphere gas before the heater element 61 generates heat is prevented. Since the heater element 61 heats the atmosphere gas whose temperature variation has been prevented, it is possible to calculate the radiation coefficient $M_1$ with high accuracy.

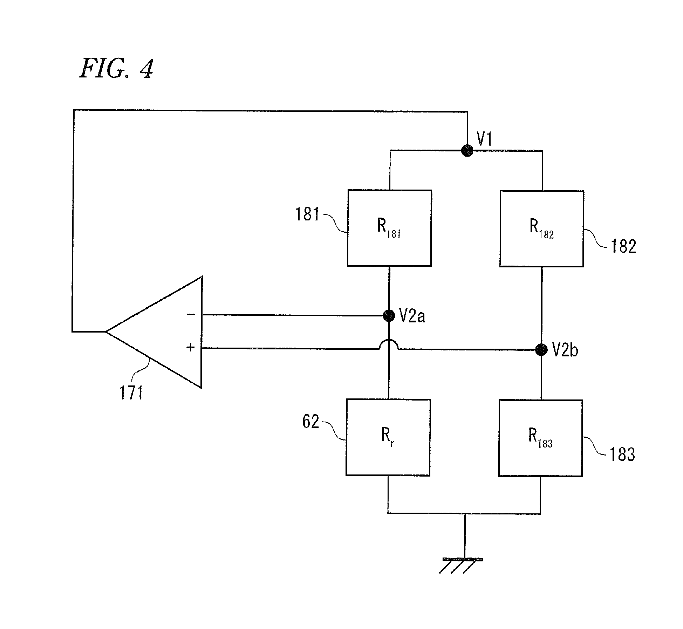

As shown in Fig. 4, the first temperature measurement element 62 shown in Figs. 1 and 2 forms, for example, a portion of a resistance bridge circuit. The resistance bridge circuit includes a resistive element 181 that is connected in series to the first temperature measurement element 62 and resistive elements 182 and 183 that are connected in parallel to the first temperature measurement element 62 and the resistive element 181. An operational amplifier 171 is connected to the resistance bridge circuit. The second temperature measurement element 63 shown in Figs. 1 and 2 also forms, for example, a portion of the resistance bridge circuit.

It is assumed that the atmosphere gas is a mixed gas and the mixed gas includes four kinds of gas components A, B, C, and D. The sum of the volume ratio $V_A$ of the gas A, the volume ratio $V_B$ of the gas B, the volume ratio $V_C$ of the gas C, and the volume ratio $V_D$ of the gas D is 1, as represented by the following Expression (17):

$$V_A + V_B + V_C + V_D = 1. \qquad \dots (17)$$

When the calorific value of the gas A per unit volume is $K_A$, the calorific value of the gas B per unit volume is $K_B$, the calorific value of the gas C per unit volume is $K_C$, and the calorific value of the gas D per unit volume is $K_D$, the calorific value Q of the mixed gas per unit volume is the sum of the values obtained by multiplying the volume ratios of the gas components by the calorific values of the gas components per unit volume. Therefore, the calorific value Q of the mixed gas per unit volume is represented by the following Expression (18):

$$Q = K_A \times V_A + K_B \times V_B + K_C \times V_C + K_D \times V_D. \qquad \dots (18)$$

The unit of the calorific value per unit volume is, for example, $MJ/m^3$.

When the radiation coefficient of the gas A is $M_A$, the radiation coefficient of the gas B is $M_B$, the radiation coefficient of the gas C is $M_C$, and the radiation coefficient of the gas D is $M_D$, the radiation coefficient $M_1$ of the mixed gas is the sum of the values obtained by multiplying the volume ratios of the gas components by the radiation coefficients of the gas components. Therefore, the radiation coefficient $M_1$ of the mixed gas is represented by the following Expression (19):

$$M_I = M_A \times V_A + M_B \times V_B + M_C \times V_C + M_D \times V_D. \qquad \dots (19)$$

Since the radiation coefficient of the gas depends on the temperature $T_H$ of the heater element 61, the radiation coefficient $M_1$ of the mixed gas is a function of the temperature $T_H$ of the heater element 61 and is represented by the following Expression (20):

$$M_I(T_H) = M_A(T_H) \times V_A + M_B(T_H) \times V_B + M_C(T_H) \times V_C + M_D(T_H) \times V_D. \qquad \dots (20)$$

Therefore, the radiation coefficient $M_{I1}(T_{H1})$ of the mixed gas when the temperature of the heater element 61 is $T_{H1}$ is represented by the following Expression (21):

$$M_{I1}(T_{H1}) = M_A(T_{H1}) \times V_A + M_B(T_{H1}) \times V_B + M_C(T_{H1}) \times V_C + M_D(T_{H1}) \times V_D. \qquad \dots (21)$$

The radiation coefficient $M_{I2}(T_{H2})$ of the mixed gas when the temperature of the heater element 61 is $T_{H2}$ is represented by the following Expression (22):

$$M_{I2}(T_{H2}) = M_A(T_{H2}) \times V_A + M_B(T_{H2}) \times V_B + M_C(T_{H2}) \times V_C + M_D(T_{H2}) \times V_D. \qquad \dots (22)$$

The radiation coefficient $M_{I3}(T_{H3})$ of the mixed gas when the temperature of the heater element 61 is $T_{H3}$ is represented by the following Expression (23):

$$M_{I3}(T_{H3}) = M_A(T_{H3}) \times V_A + M_B(T_{H3}) \times V_B + M_C(T_{H3}) \times V_C + M_D(T_{H3}) \times V_D. \quad …(22)$$

When the radiation coefficients $M_A(T_H)$, $M_B(T_H)$, $M_C(T_H)$, and $M_D(T_H)$ of the gas components have non-linearity with respect to the temperature $T_H$ of the heater element 61, Expressions (21) to (23) have a linear-independent relationship therebetween. In addition, even when the radiation coefficients $M_A(T_H)$, $M_B(T_H)$, $M_C(T_H)$, and $M_D(T_H)$ of the gas components have linearity with respect to the temperature $T_H$ of the heater element 61, Expressions (21) to (23) have a linear-independent relationship therebetween when the rates of change of the radiation coefficients $M_A(T_H)$, $M_B(T_H)$, $M_C(T_H)$, and $M_D(T_H)$ of the gas components with respect to the temperature $T_H$ of the heater element 61 are different from each other. In addition, when Expressions (21) to (23) have the linear-independent relationship therebetween, Expression 17) and Expressions (21) to (23) have the linear-independent relationship therebetween.

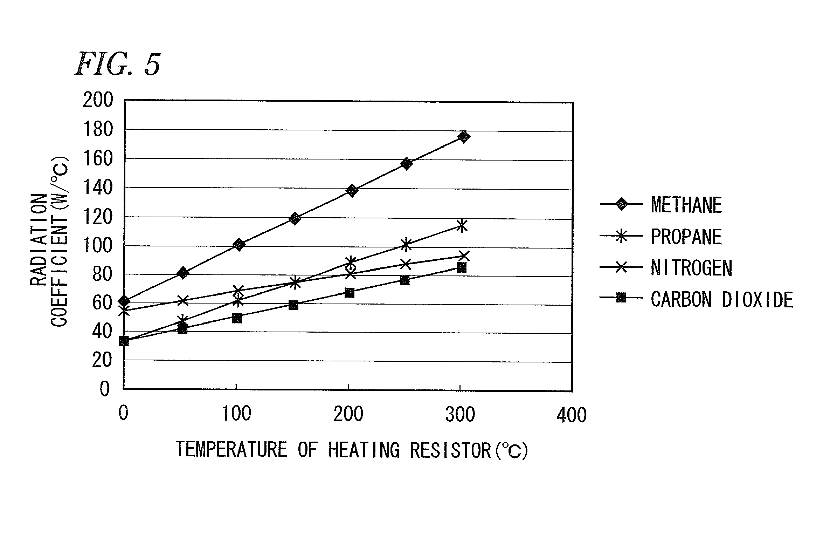

Fig. 5 is a graph illustrating the relationship between the radiation coefficients of methane ($CH_4$), propane ($C_3H_8$), nitrogen ($N_2$), and carbon dioxide ($CO_2$) included in a natural gas and the temperature of the heater element 61. The radiation coefficients of the gas components, that is, methane ($CH_4$), propane ($C_3H_8$), nitrogen ($N_2$), and carbon dioxide ($CO_2$) have linearity with respect to the temperature of the heater element 61. However, the rates of change of the radiation coefficients of methane ($CH_4$), propane ($C_3H_8$), nitrogen ($N_2$), and carbon dioxide ($CO_2$) with respect to the temperature of the heater element 61 are different from each other. Therefore, when the gas components included in the mixed gas are methane ($CH_4$), propane ($C_3H_8$), nitrogen ($N_2$), and carbon dioxide ($CO_2$), Expressions 21 to 23 have a linear-independent relationship therebetween.

The values of the radiation coefficients $M_A(T_{H1})$, $M_B(T_{H1})$, $M_C(T_{H1})$, $M_D(T_{H1})$, $M_A(T_{H2})$, $M_B(T_{H2})$, $M_C(T_{H2})$, $M_D(T_{H2})$, $M_A(T_{H3})$, $M_B(T_{H3})$, $M_C(T_{H3})$, and $M_D(T_{H3})$ of the gas components in Expressions 21 to 23 can be obtained in advance by, for example, measurement. Therefore, when Simultaneous equation 17 and Simultaneous equations (21) to (23) are solved, the volume ratio $V_A$ of the gas A, the volume ratio $V_B$ of the gas B, the volume ratio $V_C$ of the gas C, and the volume ratio $V_D$ of the gas D are respectively given as functions of the radiation coefficients $M_{I1}(T_{H1})$, $M_{I2}(T_{H2})$, and $M_{I3}(T_{H3})$ of the mixed gas, as represented by the following Expressions (24) to (27):

$$V_A = f_1[M_{I1}(T_{H1}), M_{I2}(T_{H2}), M_{I3}(T_{H3})], \qquad …(24)$$

$$V_B = f_2[M_{I1}(T_{H1}), M_{I2}(T_{H2}), M_{I3}(T_{H3})], \qquad …(25)$$

$$V_C = f_3[M_{I1}(T_{H1}), M_{I2}(T_{H2}), M_{I3}(T_{H3})], \qquad …(26)$$

$$V_D = f_4[M_{I1}(T_{H1}), M_{I2}(T_{H2}), M_{I3}(T_{H3})]. \qquad …(27)$$

In Expressions (24) to (27), n is a natural number and $f_n$ indicates a function.

Expressions (24) to (27) are substituted into Expression (18) to obtain the following Expression (28):

$$Q = K_A \times V_A + K_B \times V_B + K_C \times V_C + K_D \times V_D$$
$$= K_A \times f_1[M_{I1}(T_{H1}), M_{I2}(T_{H2}), M_{I3}(T_{H3})] + K_B \times f_2[M_{I1}(T_{H1}), M_{I2}(T_{H2}), M_{I3}(T_{H3})]$$
$$+ K_C \times f_3[M_{I1}(T_{H1}), M_{I2}(T_{H2}), M_{I3}(T_{H3})] + K_D \times f_4[M_{I1}(T_{H1}), M_{I2}(T_{H2}), M_{I3}(T_{H3})]. \quad …(28)$$

As shown in Expression (28), the calorific value Q of the mixed gas per unit volume is represented by an equation having as variables the radiation coefficients $M_{I1}(T_{H1})$, $M_{I2}(T_{H2})$, $M_{I3}(T_{H3})$ of the mixed gas when the temperature of the heater element 61 is $T_{H1}$, $T_{H2}$, and $T_{H3}$. Therefore, the calorific value Q of the mixed gas is represented by the following Expression (29) in which g indicates a function:

$$Q = g[M_{I1}(T_{H1}), M_{I2}(T_{H2}), M_{I3}(T_{H3})]. \qquad \ldots(29)$$

Therefore, the inventors found that, when Expression 29 was obtained for the mixed gas including the gas A, the gas B, the gas C, and the gas D in advance, it was possible to easily calculate the calorific value Q of a mixed gas to be examined, in which the volume ratio $V_A$ of the gas A, the volume ratio $V_B$ of the gas B, the volume ratio $V_C$ of the gas C, and the volume ratio $V_D$ of the gas D were unknown, per unit volume. Specifically, it is possible to uniquely calculate the calorific value Q of a mixed gas to be examined by measuring the radiation coefficients $M_{I1}(T_{H1})$, $M_{I2}(T_{H2})$, and $M_{I3}(TH_3)$ of the mixed gas to be examined when the temperature of the heater element 61 is $T_{H1}$, $T_{H2}$, and $T_{H3}$ and substituting the calculated values into Expression (29).

The gas components of the mixed gas are not limited to four kinds. For example, when the mixed gas includes n kinds of gas components, first, an equation having as variables the radiation coefficients $M_{I1}(T_{H1})$, $M_{I2}(T_{H2})$, $M_{I3}(T_{H3})$, ..., $M_{In-1}(T_{Hn-1})$ of the mixed gas with respect to at least n-1 kinds of temperatures $T_{H1}$, $T_{H2}$, $T_{H3}$, ..., $T_{Hn-1}$ of the heater element 61 is acquired in advance, as represented by the following Expression (30):

$$Q = g[M_{I1}(T_{H1}), M_{I2}(T_{H2}), M_{I3}(T_{H3}), ..., M_{In-1}(T_{Hn-1})], \qquad \ldots(30)$$

Then, the radiation coefficients $M_{I1}(T_{H1})$, $M_{I2}(T_{H2})$, $M_{I3}(T_{H3})$, ..., $M_{In-1}(T_{Hn-1})$ of the mixed gas to be examined, in which the volume ratio of each of the n kinds of gas components is unknown, with respect to n-1 kinds of temperatures $T_{H1}$, $T_{H2}$, $T_{H3}$, ..., $T_{HN-1}$ of the heater element 61 are measured, and the measured values are substituted into Expression (30). In this way, it is possible to uniquely calculate the calorific value Q of the mixed gas to be examined per unit volume.

However, when the mixed gas includes as a gas component alkane ($C_jH_{2j+2}$) (where j is a natural number) other than methane ($CH_4$) and propane ($C_3H_8$) in addition to methane ($CH_4$) and propane ($C_3H_8$), alkane ($C_jH_{2j+2}$) other than methane ($CH_4$) and propane ($C_3H_8$) may be regarded as a mixture of methane ($CH_4$) and propane ($C_3H_8$), which has no effect on the calculation of Expression (30). For example, Expression (30) may be calculated regarding ethane ($C_2H_6$), butane ($C_4H_{10}$), pentane ($C_5H_{12}$), and hexane ($C_6H_{12}$) as mixtures of methane ($CH_4$) and propane ($C_3H_8$) multiplied by predetermined coefficients, as represented by the following Expressions (31) to (34):

$$C_2H_6 = 0.5CH_4 + 0.5C_3H_8, \qquad \ldots(31)$$

$$C_4H_{10} = -0.5CH_4 + 1.5C_3H_8, \qquad \ldots(32)$$

$$C_5H_{12} = -1.0CH_4 + 2.0C_3H_8, \qquad \ldots(33)$$

$$C_6H_{14} = -1.5CH_4 + 2.5C_3H_8. \qquad \ldots(34)$$

When the mixed gas including n kinds of gas components includes, as gas components, z kinds (z is a natural number) of alkane ($C_jH_{2j+2}$) other than methane ($CH_4$) and propane ($C_3H_8$) in addition to methane ($CH_4$) and propane ($C_3H_8$), an equation having as variables the radiation coefficients $M_1$ of the mixed gas at n-z-1 or more kinds of temperatures may be calculated.

When the kind of gas components in the mixed gas used to calculate Expression 30 is equal to the kind of gas components in a mixed gas to be examined whose calorific value Q per unit volume is unknown, Expression (30) may be used to calculate the calorific value Q of the mixed gas to be examined. When the mixed gas to be examined includes gas components that are smaller than n kinds of gas components and gas components smaller than n kinds of gas components are included in the mixed gas used to calculate Expression (30), Expression (30) can be used. For example, when the mixed gas used to calculate Expression (30) includes four kinds of gas components, that is, methane ($CH_4$), propane ($C_3H_8$), nitrogen ($N_2$), and carbon dioxide ($CO_2$) and the mixed gas to be examined does not include nitrogen ($N_2$), but includes only three kinds of gas components, that is, methane ($CH_4$), propane ($C_3H_8$), and carbon dioxide ($CO_2$), Expression 30 can also be used to calculate the calorific value Q of the mixed gas to be examined.

When the mixed gas used to calculate Expression (30) includes as gas components methane ($CH_4$) and propane ($C_3H_8$), Expression (30) can be used even when the mixed gas to be examined includes alkane ($C_jH_{2j+2}$) which

is not included in the mixed gas used to calculate Expression (30). This is because regarding alkane ($C_jH_{2j+2}$) other than methane ($CH_4$) and propane ($C_3H_8$) as a mixture of methane ($CH_4$) and propane ($C_3H_8$) does not affect the calculation of the calorific value Q per unit volume by Expression (30), as described above.

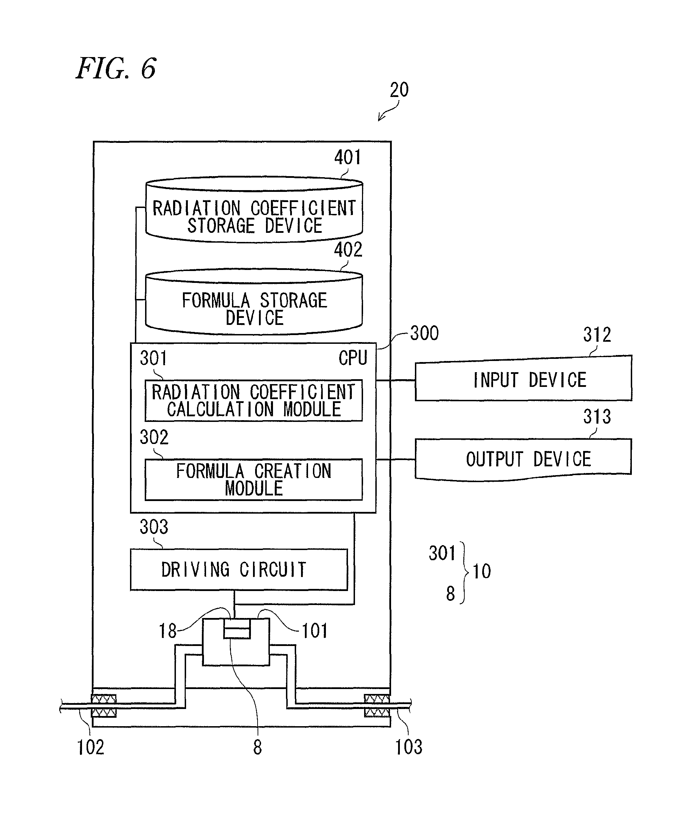

A gas property value measurement system 20 according to the first embodiment shown in Fig. 6 includes a chamber 101 that is filled up with a sample mixed gas having a known calorific value Q and a measurement mechanism 10 shown in Fig. 6 that measures a plurality of radiation coefficients $M_1$ of the sample mixed gas using the heater element 61, the first temperature measurement element 62, and the second temperature measurement element 63 shown in Figs. 1 and 2. In addition, the gas property value measurement system includes a formula creation module 302 that creates a calorific value calculation formula having the radiation coefficients $M_1$ of the gas at a plurality of temperatures of the heater element 61 as independent variables and the calorific value Q of the gas as a dependent variable, based on the known calorific value Q of the sample mixed gas and the measured values of the plurality of radiation coefficients $M_1$ of the sample mixed gas. The sample mixed gas includes plural kinds of gas components.

The measurement mechanism 10 includes the microchip 8 described with reference to Figs. 1 and 2 which is arranged in the chamber 101 into which the sample mixed gas is injected. The microchip 8 is arranged in the chamber 101 with the insulating member 18 interposed therebetween. A flow path 102 for transferring the sample mixed gas to the chamber 101 and a flow path 103 of exhausting the sample mixed gas from the chamber 101 to the outside are connected to the chamber 101.

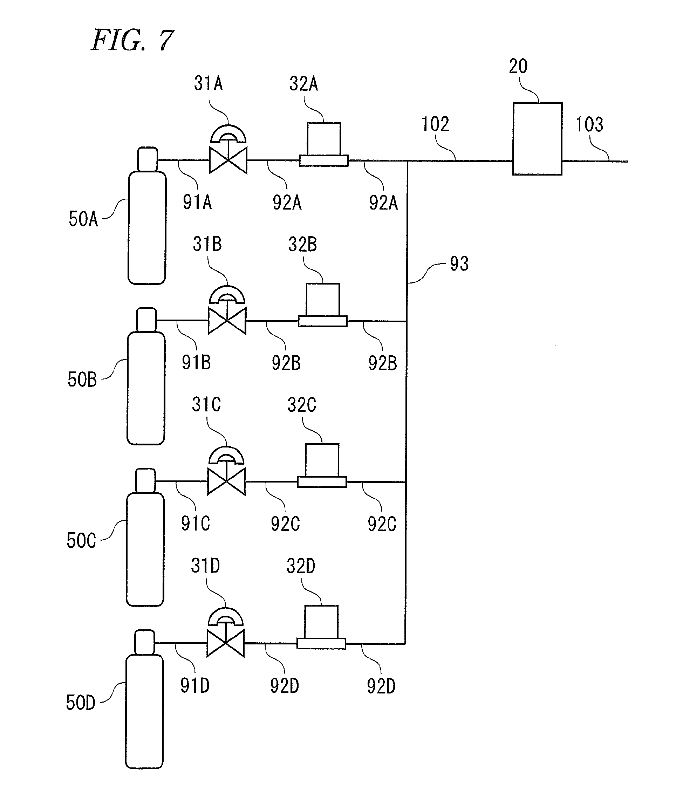

When four kinds of sample mixed gases with different calorific values Q are used, as shown in Fig. 7, a first gas cylinder 50A storing a first sample mixed gas, a second gas cylinder 50B storing a second sample mixed gas, a third gas cylinder 50C storing a third sample mixed gas, and a fourth gas cylinder 50D storing a fourth sample mixed gas are prepared. A first gas pressure adjuster 31 A for obtaining the first sample mixed gas which is adjusted to a low pressure of, for example, 0.2 MPa from the first gas cylinder 50A is connected to the first gas cylinder 50A through a flow path 91A. In addition, a first flow rate controller 32A is connected to the first gas pressure adjuster 31A through a flow path 92A. The first flow rate controller 32A controls the flow rate of the first sample mixed gas transferred to the gas property value measurement system 20 through the flow path 92A and a flow path 102.

A second gas pressure adjuster 31B is connected to the second gas cylinder 50B through a flow path 91B. In addition, a second flow rate controller 32B is connected to the second gas pressure adjuster 31B through a flow path 92B. The second flow rate controller 32B controls the flow rate of the second sample mixed gas transferred to the gas property value measurement system 20 through the flow paths 92B, 93, and 102.

A third gas pressure adjuster 31C is connected to the third gas cylinder 50C through a flow path 91C. In addition, a third flow rate controller 32C is connected to the third gas pressure adjuster 31C through a flow path 92C. The third flow rate controller 32C controls the flow rate of the third sample mixed gas transferred to the gas property value measurement system 20 through the flow paths 92C, 93, and 102.

A fourth gas pressure adjuster 31D is connected to the fourth gas cylinder 50D through a flow path 91 D. In addition, a fourth flow rate controller 32D is connected to the fourth gas pressure adjuster 31D through a flow path 92D. The fourth flow rate controller 32D controls the flow rate of the fourth sample mixed gas transferred to the gas property value measurement system 20 through the flow paths 92D, 93, and 102.

Each of the first to fourth sample mixed gases is, for example, a natural gas. Each of the first to fourth sample mixed gases includes, for example, four kinds of gas components, that is, methane ($CH_4$), propane ($C_3H_8$), nitrogen ($N_2$), and carbon dioxide ($CO_2$).

After the first sample mixed gas is filled in the chamber 101, the first temperature measurement element 62 and the second temperature measurement element 63 of the microchip 8 detect the temperature $T_1$ of the first sample mixed gas. Then, the heater element 61 is supplied with driving powers $P_{H0}$, $P_{H1}$, $P_{H2}$, and $P_{H3}$ from a driving circuit 303 shown in Fig. 6. The driving power $P_{H0}$ is weak and the temperature $T_{H0}$ of the heater element 61 shown in Figs. 1 and 2 is approximate to the temperature $T_1$ of the atmosphere gas. In contrast, when the driving powers $P_{H1}$, $P_{H2}$, and $P_{H3}$ are supplied, the heater element 61 generates heat, for example, at a temperature $T_{H1}$ of 100°C, a temperature $T_{H2}$ of 150°C, and a temperature $T_{H3}$ of 200°C.

After the first sample mixed gas is removed from the chamber 101 shown in Fig. 6, the second to fourth sample mixed gases are sequentially filled in the chamber 101. After the second to fourth sample mixed gases are filled in the chamber 101, the microchip 8 measures the temperature $T_1$ of each of the second to fourth sample mixed gases. In addition, the heater element 61 of the microchip 8 generates heat at a temperature of 100°C, 150°C, and 200°C.

When each sample mixed gas includes n kinds of gas components, the heater element 61 of the microchip 8 shown in Figs. 1 and 2 generates heat at n-1 or more kinds of different temperatures. However, as described above, alkane ($C_jH_{2j+2}$) other than methane ($CH_4$) and propane ($C_3H_8$) is regarded as a mixture of methane ($CH_4$) and propane ($C_3H_8$). Therefore, when the sample mixed gas including n kinds of gas components includes, as gas components, z kinds (z is a natural number) of alkane ($C_jH_{2j+2}$) in addition to methane ($CH_4$) and propane ($C_3H_8$), the heater element 61 generates heat at n-z-1 or more kinds of different temperatures.

The measurement mechanism 10 shown in Fig. 6 includes a radiation coefficient calculation module 301 connected to the microchip 8. As shown in Expression 14, the radiation coefficient calculation module 301 calculates the radiation coefficient $M_{I1}$ of the first sample mixed gas thermally balanced with the heater element 61 whose temperature $T_{H1}$ is 100°C, based on the temperature $T_1$ of the first sample mixed gas, the resistance value $R_{H0}$ of the heater element 61 when the temperature of the heater element 61 shown in Figs. 1 and 2 is $T_{H0}$, and the resistance value $R_{H1}$ of the heater element 61 when the temperature of the heater element 61 is $T_{H1}$ (here, 100°C). The measurement mechanism 10 also calculates the radiation coefficient $M_{I1}$ of each of the second to fourth sample mixed gases thermally balanced with the heater element 61 whose temperature $T_{H1}$ is 100°C.

As shown in Expression 15, the radiation coefficient calculation module 301 shown in Fig. 6 calculates the radiation coefficient $M_{I2}$ of the first sample mixed gas thermally balanced with the heater element 61 whose temperature $T_{H2}$ is 150°C, based on the temperature $T_1$ of the first sample mixed gas, the resistance value $R_{H0}$ of the heater element 61 when the temperature of the heater element 61 shown in Figs. 1 and 2 is $T_{H0}$, and the resistance value $R_{H2}$ of the heater element 61 when the temperature of the heater element 61 is $T_{H2}$ (here, 150°C). The measurement mechanism 10 also calculates the radiation coefficient $M_{I2}$ of each of the second to fourth sample mixed gases thermally balanced with the heater element 61 whose temperature $T_{H2}$ is 150°C.

As shown in Expression 16, the radiation coefficient calculation module 301 shown in Fig. 6 calculates the radiation coefficient $M_{I3}$ of the first sample mixed gas thermally balanced with the heater element 61 whose temperature $T_{H3}$ is 200°C, based on the temperature $T_1$ of the first sample mixed gas, the resistance value $R_{H0}$ of the heater element 61 when the temperature of the heater element 61 shown in Figs. 1 and 2 is $T_{H0}$, and the resistance value $R_{H3}$ of the heater element 61 when the temperature of the heater element 61 is $T_{H3}$ (here, 200°C). The measurement mechanism 10 also calculates the radiation coefficient $M_{I3}$ of each of the second to fourth sample mixed gases thermally balanced with the heater element 61 whose temperature $T_{H3}$ is 200°C.

The gas property value measurement system 20 shown in Fig. 6 further includes a radiation coefficient storage device 401 connected to a CPU 300. The radiation coefficient calculation module 301 stores the calculated radiation coefficients $M_{I1}$, $M_{I2}$, and $M_{I3}$ in the radiation coefficient storage device 401.

The formula creation module 302 collects, for example, the known calorific value Q of each of the first to fourth sample mixed gases, the radiation coefficient $M_{I1}$ of the gas when the temperature of the heater element 61 is 100°C, the radiation coefficient $M_{12}$ of the gas when the temperature of the heater element 61 is 150°C, and the radiation coefficient $M_{I3}$ of the gas when the temperature of the heater element 61 is 200°C. In addition, the formula creation module 302 performs multivariate analysis to calculate a calorific value calculation formula having the radiation coefficient $M_{I1}$ when the temperature of the heater element 61 is 100°C, the radiation coefficient $M_{I2}$ when the temperature of the heater element 61 is 150°C, and the radiation coefficient $M_{I3}$ when the temperature of the heater element 61 is 200°C as independent variables and the calorific values Q as dependent variables, based on the collected calorific values Q and radiation coefficients $M_{I1}$, $M_{I2}$, and $M_{I3}$.

The "multivariate analysis" includes a support vector regression and multiple regression analysis disclosed in A.J. Smola and B. Scholkopf, "A Tutorial on Support Vector Regression" (NeuroCOLT Technical Report (NC-TR-98-030), 1998) and Fuzzy Qualification Theory of Second Order disclosed in JP-A-5-141999. In addition, the radiation coefficient calculation module 301 and the formula creation module 302 are included in the central processing unit (CPU) 300.

The gas property value measurement system 20 further includes a formula storage device 402 connected to the CPU 300. The formula storage device 402 stores the calorific value calculation formula created by the formula creation module 302. An input device 312 and an output device 313 are connected to the CPU 300. For example, a keyboard and a pointing device, such as a mouse, may be used as the input device 312. For example, an image display device, such as a liquid crystal display or a monitor, and a printer may be used as the output device 313.

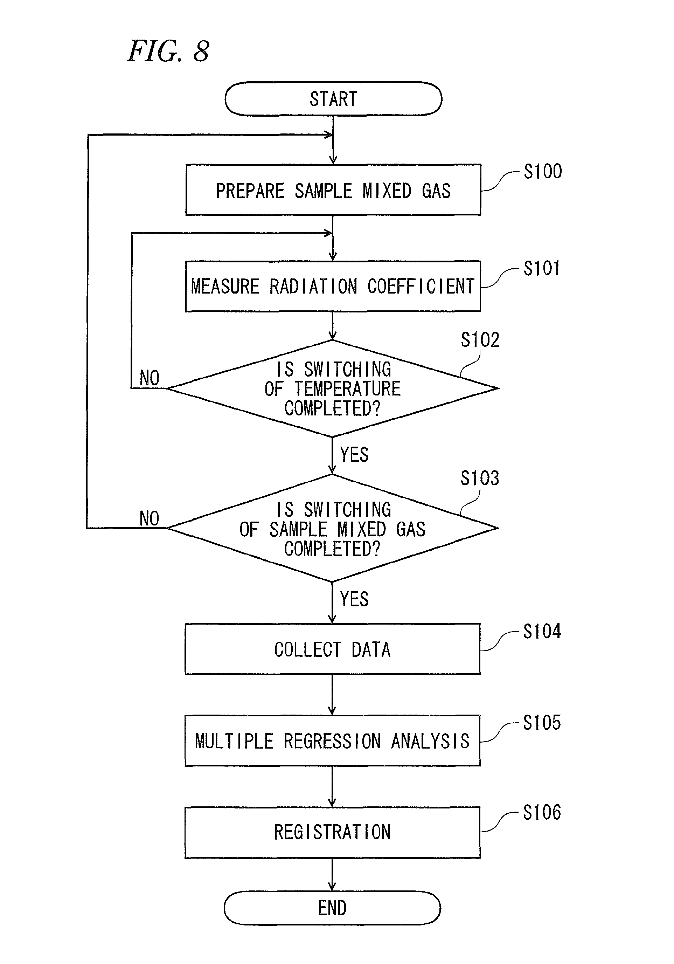

Next, a method of creating the calorific value calculation formula according to the first embodiment will be described with reference to a flowchart shown in Fig. 8.

(a) In Step S100, the valve of the first flow rate controller 32A is opened with the valves of the second to fourth flow rate controller 32B to 32D shown in Fig. 7 being closed and the first sample mixed gas is introduced into the chamber 101 shown in Fig. 6. In Step S101, the first temperature measurement element 62 and the second temperature measurement element 63 shown in Figs. 1 and 2 detect the temperature $T_1$ of the first sample mixed gas. The driving circuit 303 shown in Fig. 6 supplies the weak driving power $P_{H0}$ to the heater element 61 of the microchip 8 shown in Figs. 1 and 2. In addition, the driving circuit 303 shown in Fig. 6 supplies the driving power $P_{H1}$ to the heater element 61 shown in Figs. 1 and 2 such that the heater element 61 generates heat at 100°C. Then, the radiation coefficient calculation module 301 shown in Fig. 6 calculates the radiation coefficient $M_{I1}$ of the first sample mixed gas when the temperature of the heater element 61 is 100°C, using Expression 14. Then, the radiation coefficient calculation module 301 stores the radiation coefficient $M_{I1}$ of the first sample mixed gas when the temperature of the heater element 61 is 100°C in the radiation coefficient storage device 401.

(b) In Step S102, the driving circuit 303 determines whether the switching of the temperature of the heater element 61 shown in Figs. 1 and 2 is completed. When the switching of the temperature to 150°C and 200°C is not completed, the process returns to Step S101 and the driving circuit 303 shown in Fig. 6 controls the heater element 61 shown in Figs. 1 and 2 to generate heat at 150°C. The radiation coefficient calculation module 301 shown in Fig. 6 uses Expression 15 to calculate the radiation coefficient $M_{I2}$ of the first sample mixed gas when the temperature of the heater element 61 is 150°C and stores the calculated radiation coefficient $M_{I2}$ in the radiation coefficient storage device 401.

(c) In Step S102, it is determined again whether the switching of the temperature of the heater element 61 shown in Figs. 1 and 2 is completed. When the switching of the temperature to 200°C is not completed, the process returns to Step S101 and the driving circuit 303 shown in Fig. 6 controls the heater element 61 shown in Figs. 1 and 2 to generate heat at 200°C. The radiation coefficient calculation module 301 shown in Fig. 6 uses Expression (16) to calculate the radiation coefficient $M_{I3}$ of the first sample mixed gas when the temperature of the heater element 61 is 200°C and stores the calculated radiation coefficient $M_{I3}$ in the radiation coefficient storage device 401.

(d) When the switching of the temperature of the heater element 61 is completed, the process proceeds from Step S102 to Step S103. In Step S103, it is determined whether the switching of the sample mixed gas is completed. When the switching of the sample mixed gas to the second to fourth sample mixed gases is not completed, the process returns to Step S100. In Step S100, the valve of the first flow rate controller 32A shown in Fig. 7 is closed and the valve of the second flow rate controller 32B is opened with the valves of the third and fourth flow rate controllers 32C and 32D being closed, thereby introducing the second sample mixed gas into the chamber 101 shown in Fig. 6.

(e) Similar to the first sample mixed gas, the loop from Step S101 to Step S103 is repeatedly performed. The radiation coefficient calculation module 301 calculates the radiation coefficient $M_{I1}$ of the second sample mixed gas when the temperature of the heater element 61 is 100°C, the radiation coefficient $M_{I2}$ of the second sample mixed gas when the temperature of the heater element 61 is 150°C, and the radiation coefficient $M_{I3}$ of the second sample mixed gas when the temperature of the heater element 61 is 200°C. In addition, the radiation coefficient calculation module 301 stores the calculated radiation coefficients $M_{I1}$, $M_{I2}$, and $M_{I3}$ of the second sample mixed gas in the radiation coefficient storage device 401.

(f) Then, the loop from Step S100 to Step S103 is repeatedly performed. In this way, the radiation coefficients $M_{I1}$, $M_{I2}$, and $M_{I3}$ of the third sample mixed gas when the temperature of the heater element 61 is 100°C, 150°C, and 200°C and the radiation coefficients $M_{I1}$, $M_{I2}$, and $M_{I3}$ of the fourth sample mixed gas when the temperature of the heater element 61 is 100°C, 150°C, and 200°C are stored in the radiation coefficient storage device 401. In Step S104, the known calorific value Q of the first sample mixed gas, the known calorific value Q of the second sample mixed gas, the known calorific value Q of the third sample mixed gas, and the known calorific value Q of the fourth sample mixed gas are input from the input device 312 to the formula creation module 302. The formula creation module 302 reads the radiation coefficients $M_{I1}$, $M_{I2}$, and $M_{I3}$ of each of the first to fourth sample mixed gases when the temperature of the heater element 61 is 100°C, 150°C, and 200°C from the radiation coefficient storage device 401.

(g) In Step S105, the formula creation module 302 performs multiple regression analysis based on the calorific values Q of the first to fourth sample mixed gases and the radiation coefficients $M_{I1}$, $M_{I2}$, and $M_{I3}$ of the first to fourth sample mixed gases when the temperature of the heater element 61 is 100°C, 150°C, and 200°C. The formula creation module 302 calculates a calorific value calculation formula having the radiation coefficient $M_{I1}$ when the temperature of the heater element 61 is 100°C, the radiation coefficient $M_{I2}$ when the temperature of the heater element 61 is 150°C, and the radiation coefficient $M_{I3}$ when the temperature of the heater element 61 is 200°C as independent variables and the calorific values Q as dependent variables, using multiple regression analysis. Then, in Step S106, the formula creation module 302 stores the created calorific value calculation formula in the formula storage device 402 and ends the method of creating the calorific value calculation formula according to the first embodiment.

As described above, according to the method of creating the calorific value calculation formula of the first embodiment, it is possible to create a calorific value calculation formula capable of uniquely calculating the calorific value Q of a mixed gas to be measured.

(Second embodiment)

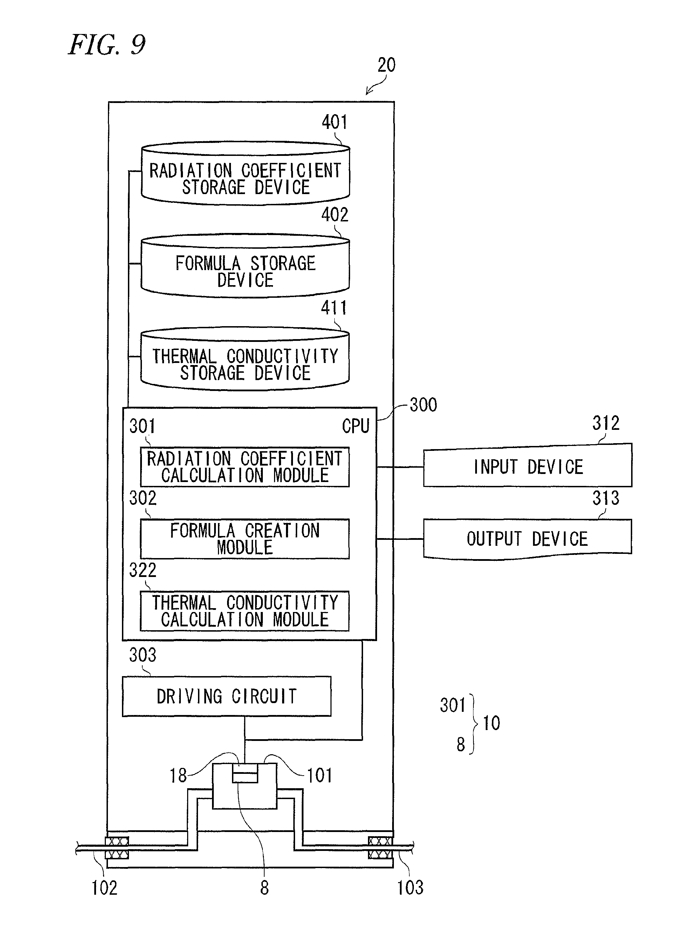

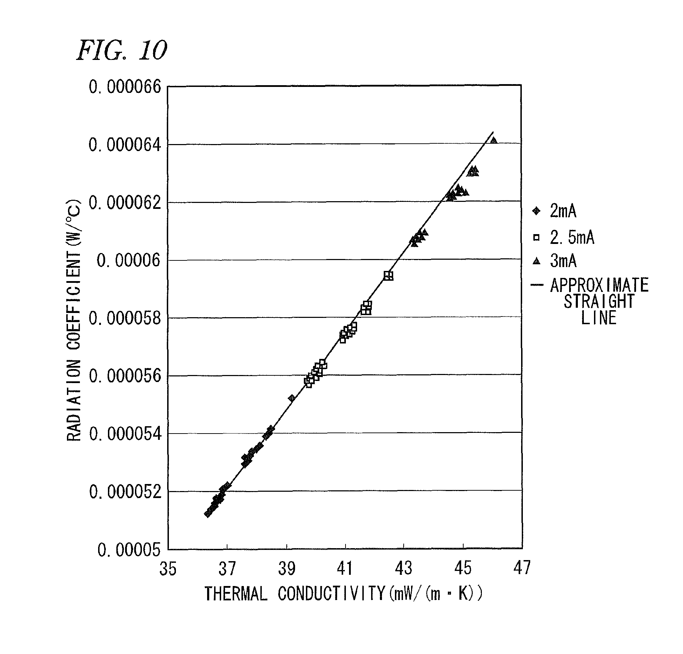

As shown in Fig. 9, a thermal conductivity storage device 411 is connected to a CPU 300 of a gas property value measurement system 20 according to a second embodiment. Fig. 10 shows the relationship between the radiation coefficient $M_1$ and thermal conductivity of a mixed gas when a current of 2 mA, 2.5 mA, and 3 mA flows to a heater element. As shown in Fig. 10, the radiation coefficient $M_1$ and thermal conductivity of the mixed gas are generally in proportion to each other. The thermal conductivity storage device 411 shown in Fig. 9 stores the correspondence between the radiation coefficient $M_1$ and thermal conductivity of the gas introduced into the chamber 101 as an approximate equation or a table in advance.

The CPU 300 according to the second embodiment further includes a thermal conductivity calculation module 322. The thermal conductivity calculation module 322 reads the radiation coefficient $M_1$ from the radiation coefficient storage device 401 and reads the correspondence between the radiation coefficient $M_1$ and thermal conductivity of the gas from the thermal conductivity storage device 411. In addition, the thermal conductivity calculation module 322 calculates the thermal conductivity of the gas introduced into the chamber 101 based on the radiation coefficient $M_1$ of the gas and the correspondence between the radiation coefficient $M_1$ and thermal conductivity of the gas.

The other components of the gas property value measurement system 20 according to the second embodiment are the same as those in the first embodiment and thus a description thereof will be omitted. According to the gas property value measurement system 20 of the second embodiment, it is possible to accurately calculate the thermal conductivity of gas based on the radiation coefficient $M_1$.

(Third embodiment)

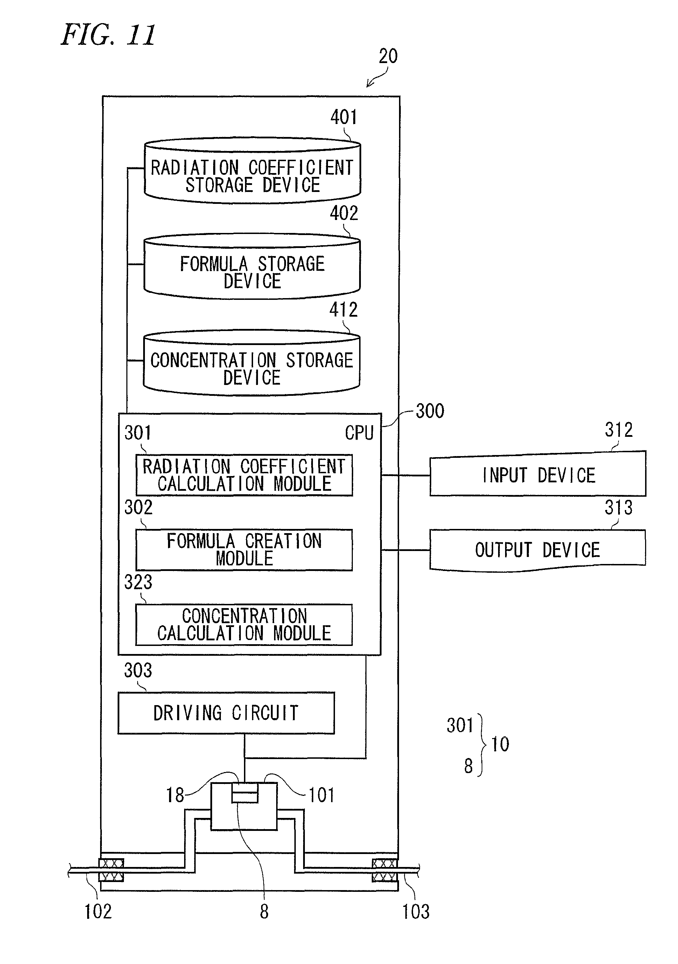

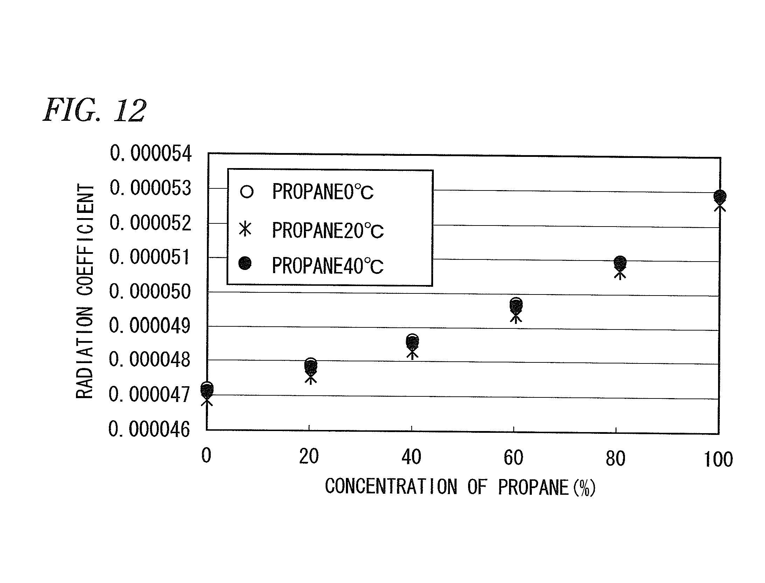

As shown in Fig. 11, a concentration storage device 412 is connected to a CPU 300 of a gas property value measurement system 20 according to a third embodiment. Fig. 12 shows the relationship between the concentration and the radiation coefficient $M_1$ of propane gas when the temperature $T_1$ of the gas is 0°C, 20°C, and 40°C. As shown in Fig. 12, the radiation coefficient $M_1$ of gas is generally in proportion to the concentration of the gas. The concentration storage device 412 shown in Fig. 11 stores the correspondence between the radiation coefficient $M_1$ and concentration of the gas introduced into the chamber 101 as, for example, an approximate equation or a table in advance.

The CPU 300 according to the third embodiment further includes a concentration calculation module 323. The concentration calculation module 323 reads the radiation coefficient $M_1$ from the radiation coefficient storage device 401 and reads the correspondence between the radiation coefficient $M_1$ and concentration of the gas from the concentration storage device 412. In addition, the concentration calculation module 323 calculates the concentration of the gas introduced into the chamber 101 based on the radiation coefficient $M_1$ of the gas and the correspondence between the radiation coefficient $M_1$ and concentration of the gas.

The other components of the gas property value measurement system 20 according to the third embodiment are the same as those in the first embodiment and thus a description thereof will be omitted. According to the gas property value measurement system 20 of the third embodiment, it is possible to accurately calculate the concentration of gas based on the radiation coefficient $M_1$ of the gas.

(Fourth embodiment)

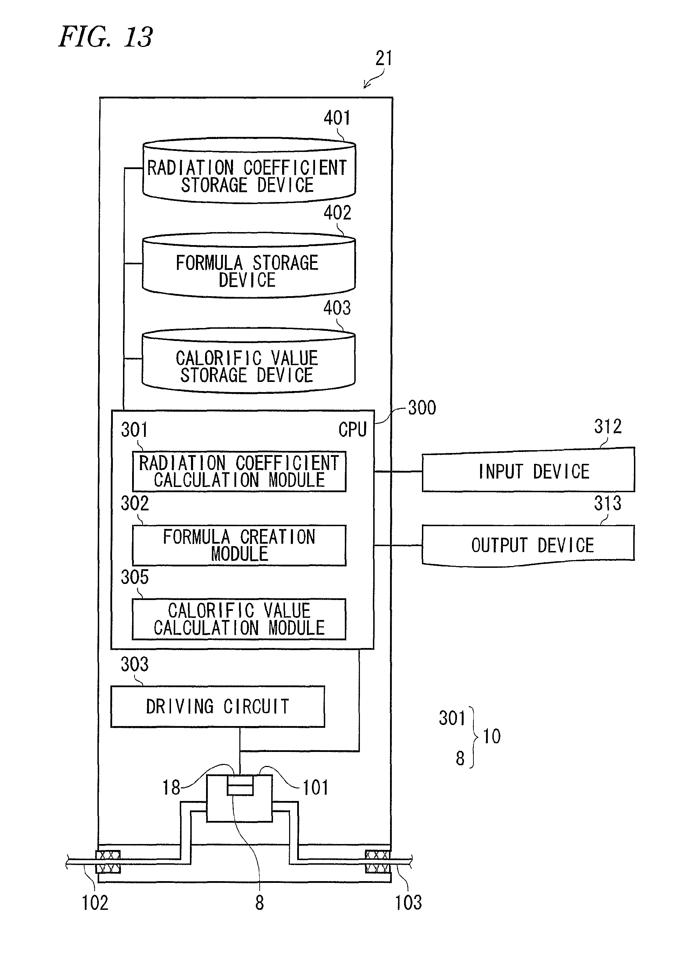

As shown in Fig. 13, a gas property value measurement system 21 according to a fourth embodiment includes a chamber 101 that is filled up with a mixed gas to be measured having an unknown calorific value Q and a measurement mechanism 10 shown in Fig. 13 that measures a plurality of radiation coefficients $M_1$ of the mixed gas to be measured using the heater element 61, the first temperature measurement element 62, and the second temperature measurement element 63 shown in Figs. 1 and 2. In addition, the gas property value measurement system 21 includes a formula storage device 402 that stores a calorific value calculation formula having the radiation coefficients $M_1$ of the gas at a plurality of temperatures of the heater element 61 as independent variables and the calorific value Q of the gas as a dependent variable and a calorific value calculation module 305 that substitutes the measured values of the radiation coefficients $M_1$ of the mixed gas to be measured at a plurality of temperatures of the heater element 61 into the independent variables of the calorific value calculation formula, which are the radiation coefficients $M_1$ of the gas with respect to a plurality of temperatures of the heater element 61, to calculate the calorific value Q of the mixed gas to be measured.

The formula storage device 402 stores the calorific value calculation formula described in the first embodiment. For example, a case in which a natural gas including methane ($CH_4$), propane ($C_3H_8$), nitrogen ($N_2$), and carbon dioxide ($CO_2$) is used as a sample mixed gas in order to create the calorific value calculation formula will be described. It is assumed that the calorific value calculation formula has the radiation coefficient $M_{I1}$ of the gas when the temperature The of the heater element 61 is 100°C, the radiation coefficient $M_{I2}$ of the gas when the temperature $T_{H2}$ of the heater element 61 is 150°C, and the radiation coefficient $M_{I3}$ of the gas when the temperature $T_{H3}$ of the heater element 61 is 200°C as independent variables.

In the fourth embodiment, for example, a natural gas that includes methane ($CH_4$), propane ($C_3H_8$), nitrogen ($N_2$), and carbon dioxide ($CO_2$) at an unknown volume ratio and has an unknown calorific value Q is introduced as a mixed gas to be measured into the chamber 101. For example, the first temperature measurement element 62 and the second temperature measurement element 63 of the microchip 8 shown in Figs. 1 and 2 detect the temperature $T_1$ of the mixed gas to be measured before the heater element 61 generates heat. The heater element 61 is supplied with driving powers $P_{H0}$, $P_{H1}$, $P_{H2}$, and $P_{H3}$ from a driving circuit 303. The driving power $P_{H0}$ is weak and the temperature $T_{H0}$ of the heater element 61 shown in Figs. 1 and 2 is approximate to the temperature $T_1$ of the mixed gas to be measured. When the driving powers $P_{H1}$, $P_{H2}$, and $P_{H3}$ are supplied, the heater element 61 generates heat, for example, at a temperature $T_{H1}$ of 100°C, a temperature $T_{H2}$ of 150°C, and a temperature $T_{H3}$ of 200°C.

As shown in Expression 14, the radiation coefficient calculation module 301 shown in Fig. 13 calculates the radiation coefficient $M_{I1}$ of the mixed gas to be measured which is thermally balanced with the heater element 61 that generates heat at 100°C. In addition, the radiation coefficient calculation module 301 calculates the radiation coefficient $M_{I2}$ of the mixed gas to be measured which is thermally balanced with the heater element 61 that generates heat at 150°C and the radiation coefficient $M_{I3}$ of the mixed gas to be measured which is thermally balanced with the heater element 61 that generates heat at 200°C. The radiation coefficient calculation module 301 stores the calculated radiation coefficients $M_{I1}$, $M_{I2}$, and $M_{I3}$ in the radiation coefficient storage device 401.

The calorific value calculation module 305 substitutes the measured values of the radiation coefficients $M_{I1}$, $M_{I2}$, and $M_{I3}$ of the mixed gas to be measured into the independent variables of the calorific value calculation formula, which are the radiation coefficients $M_{I1}$, $M_{I2}$, and $M_{I3}$ of the gas, thereby calculating the calorific value Q of the mixed gas to be measured. A calorific value storage device 403 is connected to the CPU 300. The calorific value storage device 403 stores the calorific value Q of the mixed gas to be measured which is calculated by the calorific value calculation module 305. The other components of the gas property value measurement system 21 according to the fourth embodiment are the same as those of the gas property value measurement system 20 according to the first embodiment shown in Fig. 6 and thus a description thereof will be omitted.

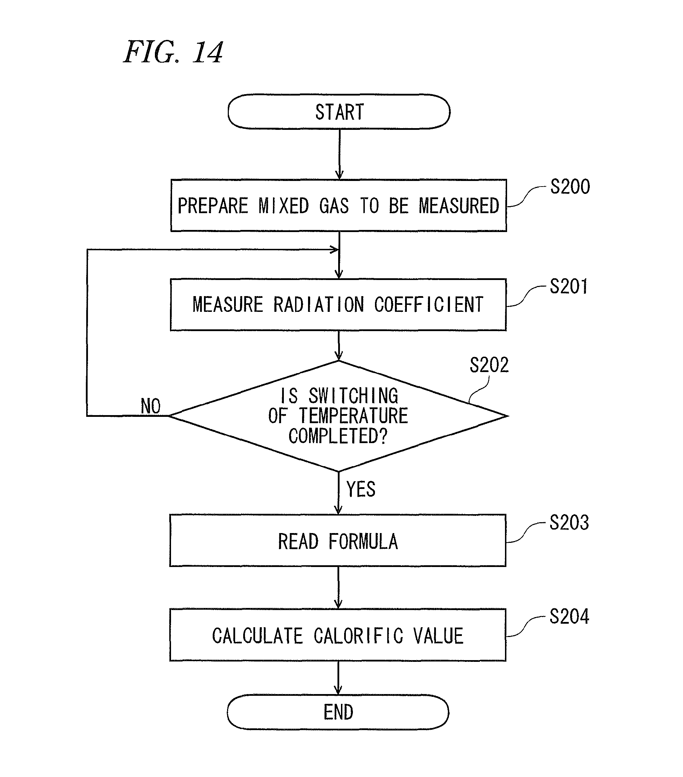

Next, a method of measuring the calorific value according to the fourth embodiment will be described with reference to a flowchart shown in Fig. 14.

(a) In Step S200, a mixed gas to be measured is introduced into the chamber 101 shown in Fig. 13. In Step S201, the first temperature measurement element 62 and the second temperature measurement element 63 shown in Figs. 1 and 2 detect the temperature $T_1$ of the mixed gas to be measured. The driving circuit 303 shown in Fig. 13 supplies the weak driving power $P_{H0}$ to the heater element 61 of the microchip 8 shown in Figs. 1 and 2. In addition, the driving circuit 303 shown in Fig. 13 supplies the driving power $P_{H1}$ to the heater element 61 shown in Figs. 1 and 2 such that the heater element 61 generates heat at 100°C. Then, the radiation coefficient calculation module 301 shown in Fig. 13 calculates the radiation coefficient $M_{I1}$ of the mixed gas to be measured when the temperature of the heater element 61 is 100°C, using Expression 14. Then, the radiation coefficient calculation module 301 stores the radiation coefficient $M_{I1}$ of the mixed gas to be measured when the temperature of the heater element 61 is 100°C in the radiation coefficient storage device 401.

(b) In Step S202, the driving circuit 303 shown in Fig. 13 determines whether the switching of the temperature of the heater element 61 shown in Figs. 1 and 2 is completed. When the switching of the temperature to 150°C and 200°C is not completed, the process returns to Step S201 and the driving circuit 303 shown in Fig. 13 controls the heater element 61 shown in Figs. 1 and 2 to generate heat at 150°C. The radiation coefficient calculation module 301 shown in Fig. 13 calculates the radiation coefficient $M_{I2}$ of the mixed gas to be measured when the temperature of the heater element 61 is 150°C and stores the calculated radiation coefficient $M_{I2}$ in the radiation coefficient storage device 401.

(c) In Step S202, it is determined again whether the switching of the temperature of the heater element 61 shown in Figs. 1 and 2 is completed. When the switching of the temperature to 200°C is not completed, the process returns to Step S201 and the driving circuit 303 shown in Fig. 13 controls the heater element 61 shown in Figs. 1 and 2 to generate heat at 200°C. The radiation coefficient calculation module 301 shown in Fig. 13 calculates the radiation coefficient $M_{I3}$ of the mixed gas to be measured when the temperature of the heater element 61 is 200°C and stores the calculated radiation coefficient $M_{I3}$ in the radiation coefficient storage device 401.

(d) When the switching of the temperature of the heater element 61 is completed, the process proceeds from Step S202 to Step S203. In Step S203, the calorific value calculation module 305 shown in Fig. 13 reads a calorific value calculation formula having the radiation coefficients $M_{I1}$, $M_{I2}$, and $M_{I3}$ of the gas when the temperature of the heater element 61 is 100°C, 150°C, and 200°C as independent variables from the formula storage device 402. In addition, the calorific value calculation module 305 reads the radiation coefficients $M_{I1}$, $M_{I2}$, and $M_{I3}$ of the mixed gas to be measured when the temperature of the heater element 61 is 100°C, 150°C, and 200°C from the radiation coefficient storage device 401.

(e) In Step S204, the calorific value calculation module 305 substitutes the radiation coefficients $M_{I1}$, $M_{I2}$, and $M_{I3}$ of the mixed gas to be measured into the independent variables of the calorific value calculation formula, which are the radiation coefficients $M_{I1}$, $M_{I2}$, and $M_{I3}$, to calculate the calorific value Q of the mixed gas to be measured. Then, the calorific value calculation module 305 stores the calculated calorific value Q in the calorific value storage device 403 and ends the method of measuring the calorific value according to the fourth embodiment.

According to the method of calculating the calorific value of the fourth embodiment, it is possible to measure the calorific value Q of a mixed gas to be measured from the measured values of the radiation coefficients $M_{I1}$, $M_{I2}$, and $M_{I3}$ of the mixed gas to be measured, without using an expensive gas chromatography device or sound speed sensor.
The percentage of carbon hydride in the natural gases varies depending on the gas field. In addition, the natural gases include, for example, nitrogen ($N_2$) or carbon dioxide ($CO_2$) in addition to carbon hydride. Therefore, the volume ratio of gas components in the natural gases varies depending on the gas field. Even when the kind of gas components is known, in many cases, the calorific value Q of the natural gas is unknown. In addition, the natural gases with different calorific values Q may be produced from the same gas field and the calorific value Q of the natural gases varies depending on the production period.
In the related art, when the a price for use of the natural gas is charged, a charging method based on the volume of the natural gas used, not the calorific value Q of the natural gas used has been used. However, it is unfair to charge for the volume of the natural gas used since the calorific value Q of the natural gas varies depending on the gas field of the natural gas. In contrast, when the method of calculating the calorific value according to the fourth embodiment is used, it is possible to easily calculate the calorific value Q of a mixed gas, such as a natural gas whose calorific value Q is unknown, since the kind of gas components is known, but the volume ratio of the gas components is unknown. Therefore, it is possible to fairly charge for the use of the mixed gas.
In the glass product manufacturing industry, when glass is heated and processed, it is preferable to supply a natural gas with a constant calorific value Q in order to maintain processing accuracy to be constant. In order to maintain processing accuracy, a technique has been examined which accurately checks the calorific values Q of the natural gases produced from a plurality of gas fields, adjusts the calorific values Q of all of the natural gases to be equal to each other, and supplies the natural gas for a process of heating and processing glass. In contrast, when the method of calculating the calorific value according to the fourth embodiment is used, it is possible to accurately check the calorific values Q of the natural gases produced from a plurality of gas fields. Therefore, it is possible to maintain the heating and processing accuracy of glass to be constant.
According to the method of calculating the calorific value of the fourth embodiment, it is possible to easily know the accurate calorific value Q of a mixed gas, such as a natural gas, and thus appropriately set the amount of air required to burn the mixed gas. Therefore, it is possible to reduce the amount of carbon dioxide ($CO_2$) emitted.

(Example 1)

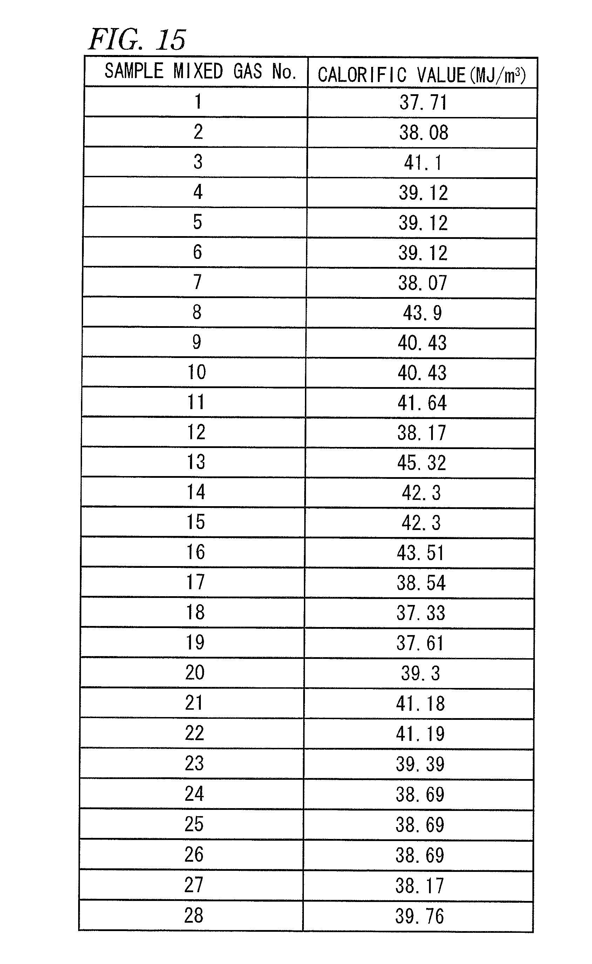

First, as shown in Fig. 15, twenty-eight kinds of sample mixed gases having known calorific values Q were prepared. Each of the twenty-eight kinds of sample mixed gases included as gas components any one or all of methane $(CH_4)$, ethane $(C_2H_6)$, propane $(C_3H_8)$, butane $(C_4H_{10})$, nitrogen $(N_2)$, and carbon dioxide $(CO_2)$. For example, sample mixed gas No. 7 included 90 vol% of methane, 3 vol% of ethane, 1 vol% of propane, 1 vol% of butane, 4 vol% of nitrogen, and 1 vol% of carbon dioxide. Sample mixed gas No. 8 included 85 vol% of methane, 10 vol% of ethane, 3 vol% of propane, and 2 vol% of butane, but did not include nitrogen and carbon dioxide. Sample mixed gas No. 9 included 85 vol% of methane, 8 vol% of ethane, 2 vol% of propane, 1 vol% of butane, 2 vol% of nitrogen, and 2 vol% of carbon dioxide.

Then, the radiation coefficient $M_I$ of each of the twenty-eight kinds of sample mixed gases was measured while the temperature of the heater element was set to 100°C, 150°C, and 200°C. For example, sample mixed gas No. 7 included six kinds of gas components. However, as described above, since ethane $(C_2H_6)$ and butane $(C_4H_{10})$ are regarded as a mixture of methane $(CH_4)$ and propane $(C_3H_8)$, the radiation coefficient $M_I$ may be measured at three kinds of temperatures. Then, a linear equation, a quadratic equation, and a cubic equation that were for calculating the calorific values Q and had the radiation coefficients $M_I$ as independent variables and the calorific values Q as dependent variables were created by a support vector regression based on the calorific values Q of the twenty-eight kinds of sample mixed gases and the measured radiation coefficients $M_I$.

When the linear equation for calculating the calorific value Q is created, three to five calibration points may be appropriately determined. The created linear equation was represented by the following Expression 35:

$$Q = 39.91 - 20.59 \times M_I(100°C) - 0.89 \times M_I(150°C) + 19.73 \times M_I(200°C). \qquad ...(35)$$

The calorific values Q of the twenty-eight kinds of sample mixed gases were calculated by Expression 35 and were compared with the true calorific values Q. As a result, the maximum error was 2.1 %.

When the quadratic equation for calculating the calorific value Q is created, eight or nine calibration points may be appropriately determined. The calorific values Q of the twenty-eight kinds of sample mixed gases were calculated by the created quadratic equation and were compared with the true calorific values Q. As a result, the maximum error was from 1.2% to 1.4%.

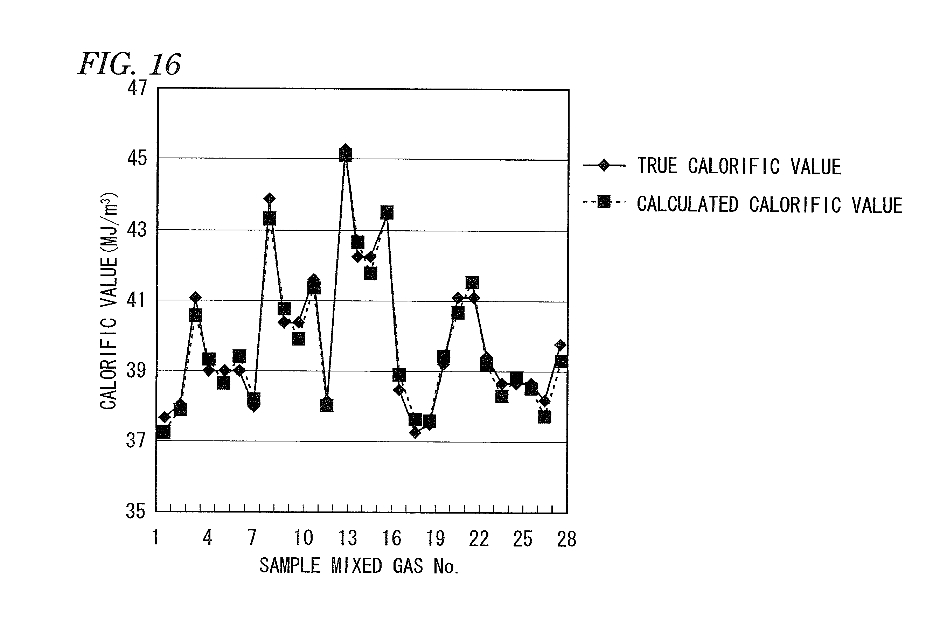

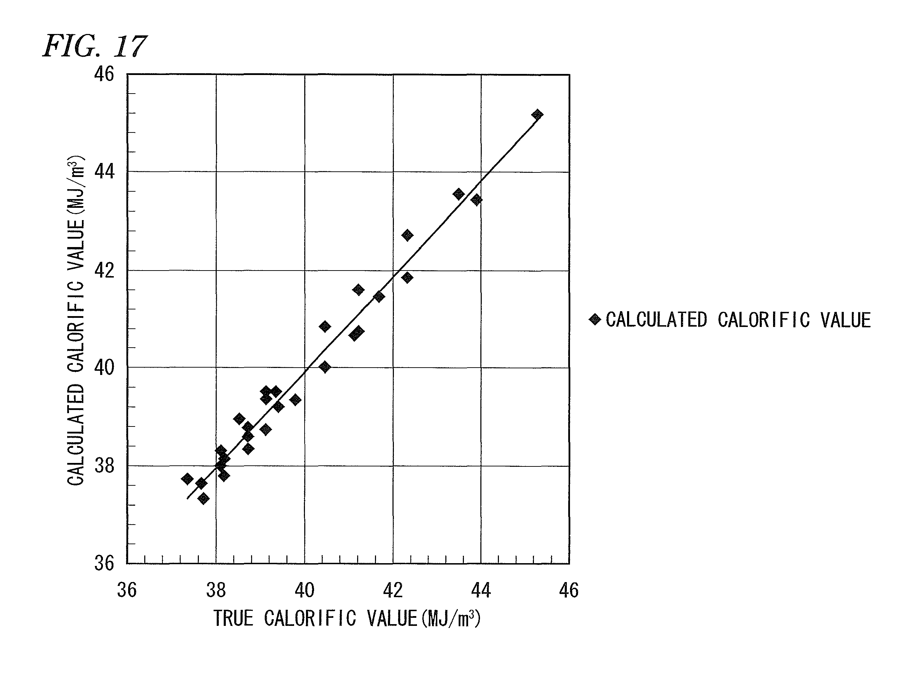

When the cubic equation for calculating the calorific value Q is created, ten to fourteenth calibration points may be appropriately determined. The calorific values Q of the twenty-eight kinds of sample mixed gases were calculated by the created cubic equation and were compared with the true calorific values Q. As a result, the maximum error was less than 1.2%. As shown in Figs. 16 and 17, the calorific values Q calculated by the cubic equation which was created using ten calibration points were approximate to the true calorific values Q.

**Claims**

1. A calorific value calculation formula creation system (21), comprising:

   a container (101) into which a mixed gas including a plurality of gases are injected;
   a heater element (61) that is provided in the container;
   a measurement mechanism (10) that corrects a drift of a resistance value of the heater element based on the ratio of a first resistance value $(R_{H0})$ of the heater element when a first temperature $(T_{H0})$ of the heater element is approximate to an atmosphere temperature $(T_I)$ and a second resistance value $(R_{H1})$ of the heater element when a second temperature $(T_{H1})$ of the heater element is more than the atmosphere temperature $(T_I)$, and measures radiation coefficients $(M_A(T_{H1}), M_B(T_{H2})...)$ of the plurality of gases at each of a plurality of heating temperatures of the heater element; and
   a formula creation module (302) that creates a calorific value calculation formula based on a given calorific value (Q) of the mixed gas and the radiation coefficients $(M_A(T_{H1}), M_B(T_{H2})...)$, wherein the calorific value calculation formula is represented by the radiation coefficients at each of the plurality of heating temperatures that are set as independent variables, and a calorific value (Q) of the mixed gas that is set as dependent variable.

2. The system of claim **1**, wherein the number of the heat temperatures is at least one less than the number of the plurality of gases.

3. The system of claim **1** or **2**, wherein the formula creation module creates the calorific value calculation formula using a support vector regression.

**4.** A method of creating a calorific value calculation formula, the method comprising:

(a) preparing a mixed gas including a plurality of gases;
(b) correcting a drift of a resistance value of a heater element (61) based on the ratio of a first resistance value $(R_{H0})$ of the heater element when a first temperature $(T_{H0})$ of the heater element is approximate to an atmosphere temperature $(T_I)$ and a second resistance value $(R_{H1})$ of the heater element when a second temperature $(T_{H1})$ of the heater element is more than the atmosphere temperature $(T_I)$;
(c) measuring radiation coefficients $(M_A(T_{H1}), M_B(T_{H2})...)$ of the plurality of gases at each of a plurality of heating temperatures of the heater element; and
(e) creating a calorific value calculation based on a given calorific value (Q) of the mixed gas and the radiation coefficients $(M_A(T_{H1}), M_B(T_{H2})...)$, wherein the calorific value calculation formula is represented by the radiation coefficients at each of the plurality of heating temperatures that are set as independent variables, and a calorific value (Q) of the mixed gas that is set as dependent variable.

**5.** The method of claim **4**,
wherein the number of heat temperatures is at least one less than the number of the plurality of gases; preferably wherein step (e) comprises: creating the calorific value calculation formula using a support vector regression.

**6.** The method of claim **4** or **5**,
wherein each of the plurality of gases is a natural gas.

**7.** A calorific value measurement system, comprising:

a container (101) into which a mixed gas including a plurality of gases are injected, wherein a calorific value of the mixed gas is unknown before measurement;
a heater element that (61) is provided in the container;
a measurement mechanism (10) that corrects a drift of a resistance value of the heater element based on the ratio of a first resistance value $(R_{H0})$ of the heater element when a first temperature $(T_{H0})$ of the heater element is approximate to an atmosphere temperature $(T_I)$ and a second resistance value $(R_{H1})$ of the heater element when a second temperature $(T_{H1})$ of the heater element is more than the atmosphere temperature $(T_I)$, and measures radiation coefficients $(M_{I1},M_{I2}...)$ of the mixed gas at a plurality of heating temperatures of the heater element;
a formula storage device that stores a calorific value calculation formula, wherein the calorific value calculation formula is represented by the radiation coefficients $(M_{I1},M_{I2}...)$ of the mixed gas that are set as independent variables, and a calorific value (Q) of the mixed gas that is set as dependent variable; and
a calorific value calculation module that substitutes the measured radiation coefficients into the radiation coefficients of the calorific value calculation formula to calculate the calorific value of the mixed gas.

**8.** The system of claim **7**,
wherein the number of the heating temperatures is at least one less than the number of the plurality of gases.

**9.** The system of claim **7** or **8**,
wherein the calorific value calculation formula is created based on calorific values of a plurality of sample mixed gases each including plural kinds of gas components and the radiation coefficients of the plurality of sample mixed gases measured at each of the plurality of the heating temperatures.

**10.** A method of measuring a calorific value comprising:

(a) preparing a mixed gas including a plurality of gases, wherein a calorific value of the mixed gas is unknown before measurement;
(b) correcting a drift of a resistance value of a heater element (61) based on the ratio of a first resistance value $(R_{H0})$ of the heater element when a first temperature $(T_{H0})$ of the heater element is approximate to an atmosphere temperature $(T_I)$ and a second resistance value $(R_{H1})$ of the heater element when a second temperature $(T_{H1})$ of the heater element is more than the atmosphere temperature $(T_I)$;
(c) measuring radiation coefficients $(M_{I1},M_{I2}...)$ of the mixed gas at a plurality of heating temperatures of the heater element;

(d) preparing a calorific value calculation formula, wherein the calorific value calculation formula is represented by the radiation coefficients ($M_{I1}, M_{I2}...$) of the mixed gas that are set as independent variables, and a calorific value (Q) of the mixed gas that is set as dependent variable; and

(e) substituting the measured radiation coefficients into the radiation coefficients of the calorific value calculation formula to calculate the calorific value of the mixed gas.

11. The method of claim **10**,

wherein the number of the heating temperatures is at least one less than the number of the plurality of gases.

12. The method of claim **10** or **11**,

wherein, in step (d), the calorific value calculation formula is created based on calorific values of a plurality of sample mixed gases each including plural kinds of gas components and the radiation coefficients of the plurality of sample mixed gases measured at each of the plurality of the heating temperatures.

13. A property measurement system comprising:

a container (101) into which a gas is injected;
a heater element (61) that is provided in the container; and
a measurement mechanism (10) that corrects a drift of a resistance value of the heater element based on the ratio of a first resistance value ($R_{H0}$) of the heater element when a first temperature ($T_{H0}$) of the heater element is approximate to an atmosphere temperature ($T_I$) and a second resistance value ($R_{H1}$) of the heater element when a second temperature ($T_{H1}$) of the heater element is more than the atmosphere temperature ($T_I$), and measures a radiation coefficient (M) of the gas.

14. The system of claim **13**, further comprising:

a thermal conductivity calculation module that calculates a thermal conductivity of the gas based on the measured radiation coefficient

15. A method of measuring a property, the method comprising:

(a) preparing a gas;
(b) correcting a drift of a resistance value of a heater element (61) based on the ratio of a first resistance value ($R_{H0}$) of the heater element when a first temperature ($T_{H0}$) of the heater element is approximate to an atmosphere temperature ($T_I$) and a second resistance value ($R_{H1}$) of the heater element when a second temperature ($T_{H1}$) of the heater element is more than the atmosphere temperature ($T_I$); and
(c) measuring a radiation coefficient of the gas.

## FIG. 1

*FIG. 2*

*FIG. 3*

$V_{in}$ (5. 0V)

162

Sw1

$V_{L3}$ (3. 4V)

163

170

Sw2

$V_{L2}$ (2. 4V)

$V_{out}$

+

164

−

Sw3

$V_{L1}$ (1. 5V)

160

165

Sw4

61

$V_{L0}$ (0. 2V)

166

*FIG. 4*

FIG. 5

*FIG. 6*

20

401

RADIATION COEFFICIENT
STORAGE DEVICE

402

FORMULA STORAGE
DEVICE

300

301                  CPU

RADIATION COEFFICIENT
CALCULATION MODULE

302

FORMULA CREATION
MODULE

312

INPUT DEVICE

313

OUTPUT DEVICE

303

DRIVING CIRCUIT

301
   }10
8

18        101

102             8            103

## FIG. 7

*FIG. 8*

```
        ┌──────────────────┐
        │      START       │
        └──────────────────┘
                 │
    ┌────────────┘
    │            ▼
    │   ┌──────────────────────────┐
    │   │ PREPARE SAMPLE MIXED GAS │────S100
    │   └──────────────────────────┘
    │            │
    │   ┌────────┘
    │   │        ▼
    │   │   ┌──────────────────────────────┐
    │   │   │ MEASURE RADIATION COEFFICIENT│────S101
    │   │   └──────────────────────────────┘
    │   │            │
    │   │            ▼                         S102
    │   │         ╱──────────────╲
    │   │  NO    ╱  IS SWITCHING  ╲
    │   └───────<   OF TEMPERATURE >
    │           ╲   COMPLETED?    ╱
    │            ╲──────────────╱
    │                  │ YES
    │                  ▼                       S103
    │               ╱──────────────╲
    │   NO         ╱  IS SWITCHING  ╲
    └─────────────<  OF SAMPLE MIXED >
                  ╲  GAS COMPLETED? ╱
                   ╲──────────────╱
                        │ YES
                        ▼
              ┌──────────────────────────┐
              │      COLLECT DATA        │────S104
              └──────────────────────────┘
                        │
                        ▼
              ┌──────────────────────────┐
              │ MULTIPLE REGRESSION ANALYSIS │────S105
              └──────────────────────────┘
                        │
                        ▼
              ┌──────────────────────────┐
              │      REGISTRATION        │────S106
              └──────────────────────────┘
                        │
                        ▼
              ┌──────────────────────────┐
              │          END             │
              └──────────────────────────┘
```

*FIG. 9*

20

401
RADIATION COEFFICIENT
STORAGE DEVICE

402
FORMULA STORAGE
DEVICE

411
THERMAL CONDUCTIVITY
STORAGE DEVICE

300
CPU

301
RADIATION COEFFICIENT
CALCULATION MODULE

302
FORMULA CREATION
MODULE

322
THERMAL CONDUCTIVITY
CALCULATION MODULE

312
INPUT DEVICE

313
OUTPUT DEVICE

303
DRIVING CIRCUIT

301
8 } 10

18    101

102        8        103

FIG. 10

*FIG. 11*

FIG. 12

## FIG. 13

**21**

- **401** RADIATION COEFFICIENT STORAGE DEVICE
- **402** FORMULA STORAGE DEVICE
- **403** CALORIFIC VALUE STORAGE DEVICE

**300** CPU

- **301** RADIATION COEFFICIENT CALCULATION MODULE
- **302** FORMULA CREATION MODULE
- **305** CALORIFIC VALUE CALCULATION MODULE

**312** INPUT DEVICE

**313** OUTPUT DEVICE

**303** DRIVING CIRCUIT

**18** **101**

301 } 10
8 }

**102** **8** **103**

EP 2 369 337 A2

*FIG. 14*

```
                    ┌─────────────┐
                    │    START    │
                    └──────┬──────┘
                           │
                           ▼
         ┌──────────────────────────────────┐
         │ PREPARE MIXED GAS TO BE MEASURED  │──── S200
         └──────────────────────────────────┘
                           │
              ┌────────────▼──────────────┐
              │ MEASURE RADIATION COEFFICIENT │──── S201
              └───────────────┬────────────┘
                              │
                              ▼
                         ◇ S202
              NO    IS SWITCHING
          ◄────── OF TEMPERATURE
                   COMPLETED?
                              │ YES
                              ▼
              ┌─────────────────────────┐
              │      READ FORMULA        │──── S203
              └────────────┬────────────┘
                           │
                           ▼
              ┌─────────────────────────┐
              │ CALCULATE CALORIFIC VALUE│──── S204
              └────────────┬────────────┘
                           │
                           ▼
                    ┌─────────────┐
                    │     END     │
                    └─────────────┘
```

*FIG. 15*

| SAMPLE MIXED GAS No. | CALORIFIC VALUE (MJ/m³) |
|---|---|
| 1 | 37.71 |
| 2 | 38.08 |
| 3 | 41.1 |
| 4 | 39.12 |
| 5 | 39.12 |
| 6 | 39.12 |
| 7 | 38.07 |
| 8 | 43.9 |
| 9 | 40.43 |
| 10 | 40.43 |
| 11 | 41.64 |
| 12 | 38.17 |
| 13 | 45.32 |
| 14 | 42.3 |
| 15 | 42.3 |
| 16 | 43.51 |
| 17 | 38.54 |
| 18 | 37.33 |
| 19 | 37.61 |
| 20 | 39.3 |
| 21 | 41.18 |
| 22 | 41.19 |
| 23 | 39.39 |
| 24 | 38.69 |
| 25 | 38.69 |
| 26 | 38.69 |
| 27 | 38.17 |
| 28 | 39.76 |

FIG. 16

EP 2 369 337 A2

*FIG. 17*

**EP 2 369 337 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004514138 T

- JP 5141999 A

37